Europäisches Patentamt

European Patent Office

Office européen des brevets

(19)

(11) **EP 1 159 247 B1**

(12) **EUROPÄISCHE PATENTSCHRIFT**

(45) Veröffentlichungstag und Bekanntmachung des Hinweises auf die Patenterteilung:
**15.10.2003 Patentblatt 2003/42**

(51) Int Cl.$^7$: **C07C 51/25**, C07C 45/35, C07C 57/04, C07C 47/22

(21) Anmeldenummer: **00912516.2**

(22) Anmeldetag: **28.02.2000**

(86) Internationale Anmeldenummer:
**PCT/EP00/01629**

(87) Internationale Veröffentlichungsnummer:
**WO 00/053557 (14.09.2000 Gazette 2000/37)**

(54) **VERFAHREN DER KATALYTISCHEN GASPHASENOXIDATION VON PROPEN ZU ACRYLSÄURE**

METHOD FOR THE CATALYTIC GAS PHASE OXIDATION OF PROPENE INTO ACRYLIC ACID

PROCEDE D'OXYDATION CATALYTIQUE EN PHASE GAZEUSE DE PROPENE POUR FORMER DE L'ACIDE ACRYLIQUE

(84) Benannte Vertragsstaaten:
**BE DE ES FR IT**

(30) Priorität: **10.03.1999 DE 19910506**
**10.03.1999 DE 19910508**
**17.06.1999 DE 19927624**
**07.10.1999 DE 19948241**
**07.10.1999 DE 19948248**
**08.10.1999 DE 19948523**

(43) Veröffentlichungstag der Anmeldung:
**05.12.2001 Patentblatt 2001/49**

(73) Patentinhaber: **BASF AKTIENGESELLSCHAFT**
**67056 Ludwigshafen (DE)**

(72) Erfinder:
• **UNVERRICHT, Signe**
**D-68169 Mannheim (DE)**
• **ARNOLD, Heiko**
**D-67056 Ludwigshafen (DE)**
• **TENTEN, Andreas**
**D-67487 Maikammer (DE)**
• **HAMMON, Ulrich**
**D-68165 Mannheim (DE)**
• **NEUMANN, Hans-Peter**
**D-67067 Ludwigshafen (DE)**
• **HARTH, Klaus**
**D-67317 Altleiningen (DE)**

(56) Entgegenhaltungen:
**EP-A- 0 184 790         EP-A- 0 575 897**
**DE-A- 3 113 179         DE-A- 4 442 346**

EP 1 159 247 B1

Printed by Jouve, 75001 PARIS (FR)

## Beschreibung

Vorliegende Erfindung betrifft ein verfahren der katalytischen Gasphasenoxidation von Propen zu Acrylsäure, bei dem man ein Propen, molekularen Sauerstoff und wenigstens ein Inertgas enthaltendes Reaktionsgasausgangs- gemisch 1, das den molekularen Sauerstoff und das Propen in einem molaren Verhältnis $O_2 : C_3H_6 \geq 1$, enthält, zu- nächst in einer ersten Reaktionsstufe bei erhöhter Temperatur so über einen ersten Festbettkatalysator, dessen Aktivmasse wenigstens ein Molybdän und/oder Wolfram sowie Wismut, Tellur, Antimon, Zinn und/oder Kupfer enthaltendes Multimetalloxid ist, führt, daß der Propenumsatz bei einmaligem Durchgang ≥ 90 mol-% und die damit einhergehende Selektivität der Acroleinbildung sowie der Acrylsäurenebenproduktbildung zusammengenommen ≥ 90 mol-% betragen, die Temperatur des die erste Reaktionsstufe verlassenden Produktgasgemisches durch direkte und/oder indirekte Kühlung gegebenenfalls verringert und dem Produktgasgemisch gegebenenfalls molekularen Sauerstoff und/ oder Inertgas zugibt, und danach das Produktgasgemisch als Acrolein, molekularen Sauerstoff und wenigstens ein Inertgas enthaltendes Reaktionsgasausgangsgemisch 2, das den molekularen Sauerstoff und das Acrolein in einem molaren Verhältnis $O_2 : C_3H_4O \geq 0{,}5$ enthält, in einer zweiten Reaktionsstufe bei erhöhter Temperatur so über einen zweiten Festbettkatalysator, dessen Aktivmasse wenigstens ein Molybdän und Vanadin enthaltendes Multimetalloxid ist, führt, daß der Acroleinumsatz bei einmaligem Durchgang ≥ 90 mol-% und die Selektivität der über beide Reaktionsstufen bilanzierten Acrylsäurebildung, bezogen auf umgesetztes Propen, ≥ 80 mol-% beträgt.

Das vorgenannte Verfahren der katalytischen Gasphasenoxidation von Propen zu Acrylsäure ist allgemein bekannt (vgl. z.B. DE-A 3002829). Im besonderen sind die beiden Reaktionsstufen für sich bekannt (vgl. z.B. EP-A 714700, EP-A 700893, EP-A 15565, DE-C 2830765, DE-C 3338380, JP-A 91/294239, EP-A 807465, WO 98/24746, EP-B 279374, DE-C 2513405, DE-A 3300044, EP-A 575897 und DE-A 19855913).

Acrylsäure ist ein bedeutendes Monomeres, das als solches oder in Form seiner Alkylester zur Erzeugung von z.B. als Klebstoffen geeigneten Polymerisaten Verwendung findet.

Die Zielsetzung einer jeden zweistufigen Festbettgasphasenoxidation von Propen zu Acrylsäure besteht grundsätzlich darin, eine möglichst hohe Raum-Zeit-Ausbeute an Acrylsäure ($RZA_{AS}$) zu erzielen (das ist bei einer kontinuierlichen Verfahrensweise die je Stunde und Gesamtvolumen der verwendeten Katalysatorschüttung in Litern erzeugte Gesamtmenge an Acrylsäure).

Es besteht deshalb generelles Interesse daran, eine solche zweistufige Festbettgasphasenoxidation von Propen zu Acrylsäure einerseits unter einer möglichst hohen Belastung der ersten Festbettkatalysatorschüttung mit Propen (darunter wird die Menge an Propen in Normlitern (= Nl; das Volumen in Liter, das die entsprechende Propenmenge bei Normalbedingungen, d.h., bei 25°C und 1 bar, einnehmen würde) verstanden, die als Bestandteil des Reaktionsgasausgangsgemisches 1 pro Stunde durch einen Liter an Katalysatorschüttung 1 geführt wird) und andererseits unter einer möglichst hohen Belastung der zweiten Festbettkatalysatorschüttung mit Acrolein (darunter wird die Menge an Acrolein in Normlitern (= Nl; das Volumen in Liter, das die entsprechende Acroleinmenge bei Normalbedingungen, d. h., bei 25°C und 1 bar, einnehmen würde) verstanden, die als Bestandteil das Reaktionsgemisches 2 pro Stunde durch einen Liter an Katalysatorschüttung 2 geführt wird) durchzuführen, ohne dabei den beim einmaligen Durchgang der beiden Reaktionsgasausgangsgemische 1, 2 durch die beiden Festbettkatalysatorschüttungen erfolgenden Umsatz an Propen und Acrolein sowie die über beide Reaktionsstufen bilanzierte Selektivität der damit einhergehenden Acrylsäurebildung (bezogen auf umgesetztes Propen) nennenswert zu beeinträchtigen.

Die Realisierung des Vorgenannten wird durch die Tatsache beeinträchtigt, daß sowohl die Festbettgasphasenoxidation von Propen zu Acrolein als auch die Festbettgasphasenoxidation von Acrolein zu Acrylsäure einerseits stark exotherm verläuf t und andererseits von einer Vielfalt möglicher Parallel- und Folgereaktionen begleitet wird.

Prinzipiell können bei der eingangs beschriebenen Festbettgasphasenoxidation von Propen zu Acrylsäure die als katalytische Aktivmassen geeigneten Multimetalloxide in Pulverform eingesetzt werden. Üblicherweise werden sie jedoch zu bestimmten Katalysatorgeometrien geformt eingesetzt, da Aktivmassenpulver wegen des schlechten Gasdurchgangs für eine großtechnische Verwendung ungeeignet ist.

Die Aufgabe der vorliegenden Erfindung bestand nun darin, die Geometrie der in den beiden Katalysatorfestbetten der eingangs beschriebenen katalytischen Festbettgasphasenoxidation zu verwendenden Katalysatorformkörper so zu wählen, daß bei hoher Eduktbelastung der Festbettkatalysatorschüttungen und vorgegebenem Eduktumsatz bei einmaligem Durchgang durch die Festbettkatalysatorschüttungen eine möglichst hohe Selektivität der Zielverbindung Acrylsäure resultiert.

Dabei konnte von nachfolgendem Stand der Technik ausgegangen werden

Die konventionellen Verfahren der katalytischen Festbettgasphasenoxidation von Propen zu Acrolein bzw. von Acrolein zu Acrylsäure, die dadurch charakterisiert sind, daß als ein Hauptbestandteil des inerten Verdünnungsgases Stickstoff und außerdem ein in einer Reaktionszone befindlicher und längs dieser Reaktionszone homogener, d.h., über die Festbettkatalysatorschüttung chemisch einheitlich zusammengesetzter, Festbettkatalysator verwendet und die Temperatur der Reaktionszone auf einem über die Reaktionszone einheitlichen Wert gehalten wird (unter Temperatur einer Reaktionszone wird hier die Temperatur der in der Reaktionszone befindlichen Festbettkatalysatorschüttung bei Ausübung des Verfahrens in Abwesenheit einer chemischen Reaktion verstanden; ist diese Temperatur innerhalb der Reaktionszone nicht konstant, so meint der Begriff Temperatur einer Reaktionszone hier den Zahlenmittelwert der Temperatur der Katalysatorschüttung längs der Reaktionszone), beschränken den anzuwendenden Wert der Propen- bzw. Acroleinbelastung der Festbettkatalysatorschüttung üblicherweise auf vergleichsweise niedrige Werte.

So liegt der angewandte Wert der Propenbelastung der Festbettkatalysatorschüttung normalerweise bei Werten ≤ 155 Nl Propen/l Katalysatorschüttung ·h (vgl. z.B. EP-A 15565 (maximale Propenlast = 120 Nl Propen/l ·h), DE-C 2830765 (maximale Propenlast = 94,5 Nl Propen/l · h), EP-A 804465 (maximale Propenlast = 128 Nl Propen/l · h), EP-B 279374 (maximale Propenlast = 112 Nl Propen/l · h), DE-C 2513405 (maximale Propenlast = 110 Nl Propen/l · h), DE-A 3300044 (maximale Propenlast = 112 Nl Propen/l · h) und EP-A 575897 (maximale Propenlast = 120 Nl Propen/l · h).

Ebenso beträgt in im wesentlichen allen Beispielen der DE-C 3338380 die maximale Propenlast 126 Nl Propen/l · h; lediglich in Beispiel 3 dieser Schrift wird eine Propenlast von 162 Nl/l · h realisiert, wobei als Katalysatorformkörper ausschließlich aus Aktivmasse bestehende Vollzylinder mit einer Länge von 7 mm und einem Durchmesser von 5 mm verwendet werden.

In der EP-B 450596 wird unter Anwendung einer strukturierten. Katalysatorschüttung bei ansonsten konventioneller Verfahrensweise eine Propenlast der Katalysatorschüttung von 202,5 Nl/Propen/l · h offenbart. Als Katalysatorformkörper werden kugelförmige Schalenkatalysatoren eingesetzt.

In der EP-A 293224 werden ebenfalls Propenbelastungen oberhalb von 160 Nl Propen/l · h offenbart, wobei ein an molekularem Stickstoff vollkommen freies inertes Verdünnungsgas verwendet wird. Die angewendete Katalysatorgeometrie wird dabei nicht mitgeteilt.

Die EP-A 253409 und das zugehörige Äquivalent, die EP-A 257565, offenbaren, daß bei Verwendung eines inerten Verdünnungsgases, das eine höhere molare Wärmekapazität als molekularer Stickstoff aufweist, der Anteil an Propen im Reaktionsgasausgangsgemisch erhöht werden kann. Nichtsdestotrotz liegt aber auch in den beiden vorgenannten Schriften die maximale realisierte Propenbelastung der Katalysatorschüttung bei 140 Nl Propen/l · h. Zur anzuwendenden Katalysatorgeometrie machen beide Schriften keine Aussage.

In entsprechender Weise wie die konventionellen Verfahren der katalytischen Festbettgasphasenoxidation von Propen zu Acrolein beschränken auch die konventionellen Verfahren der katalytischen Festbettgasphasenoxidation von Acrolein zu Acrylsäure die Acroleinbelastung der Festbettkatalysatorschüttung normalerweise auf Werte ≤ 150 Nl Acrolein/l Katalysator · h (vgl. z.B. EP-B 700893; als Katalysatorformkörper werden kugelförmige Schalenkatalysatoren eingesetzt).

Zweistufige Gasphasenoxidationen von Propen zu Acrylsäure, bei denen in den beiden Oxidationsstufen sowohl eine hohe Propenbelastung als auch eine hohe Acroleinbelastung des jeweiligen Festbettkatalysators gefahren werden, sind aus dem Stand der Technik so gut wie nicht bekannt.

Eine der wenigen Ausnahmen bilden die bereits zitierte EP-A 253409 und das zugehörige Äquivalent, die EP-A 257565. Eine weitere Ausnahme bildet die bereits zitierte EP-A 293224, wobei in der Stufe der oxidation des Acroleins kugelförmige Schalenkatalysatoren verwendet werden.

Die prinzipielle Verwendbarkeit von Ringen als Geometrie für die in den beiden relevanten Oxidationsstufen einzusetzenden Katalysatorformkörper ist aus dem Stand der Technik bekannt.

Beispielsweise wird die Ringgeometrie in der DE-A 3113179 unter dem Aspekt "möglichst geringer Druckverlust" ganz generell für exotherme Festbettgasphasenoxidationen nahegelegt. Allerdings weist diese Schrift daraufhin, daß der Einfluß der Geometrie auf die Selektivität der Produktbildung von Reaktion zu Reaktion unterschiedlich sein kann.

Im übrigen ist die Verwendung von Ringgeometrie aufweisenden Katalysatorformkörpern für die katalytische Gasphasenoxidation von Propen und/oder Acrolein z.B. aus der EP-A 575897, der DE-A 19855913 und der EP-A 700893 vorbekannt. In allen Fällen war die Anwendung der Ringgeometrie aufweisenden Katalysatorformkörper jedoch auf geringe Eduktbelastungen beschränkt.

Gegenstand der vorliegenden Erfindung ist daher ein Verfahren der katalytischen Gasphasenoxidation von Propen zu Acrylsäure, bei. dem man ein Propen, molekularen Sauerstoff und wenigstens ein Inertgas enthaltendes Reaktionsgasausgangsgemisch 1, das den molekularen Sauerstoff und das Propen in einem molaren Verhältnis $O_2$ : $C_3H_6 \geq 1$ enthält, zunächst in einer ersten Reaktionsstufe bei erhöhter Temperatur so über einen ersten Festbettkatalysator, dessen Aktivmasse wenigstens ein Molybdän und/oder Wolfram sowie Wismut, Tellur, Antimon, Zinn und/oder Kupfer enthaltendes Multimetalloxid ist, führt, daß der Propenumsatz bei einmaligem Durchgang ≥ 90 mol-% und die damit einhergehende Selektivität der Acroleinbildung sowie der Acrylsäurenebenproduktbildung zusammengenommen ≥ 90 mol-% betragen, die Temperatur des die erste Reaktionsstufe verlassenden Produktgasgemisches durch direkte und/oder indirekte Kühlung gegebenenfalls verringert und dem Produktgasgemisch gegebenenfalls molekularen Sauerstoff und/oder Inertgas zugibt, und danach das Produktgasgemisch als Acrolein, molekularen Sauerstoff und wenigstens ein Inertgas enthaltendes Reaktionsgasausgangsgemisch 2, das den molekularen Sauerstoff und das Acrolein in einem molaren Verhältnis $O_2 : C_3H_4O \geq 0,5$ enthält, in einer zweiten Reaktionsstufe bei erhöhter Temperatur so über einen zweiten Festbettkatalysator, dessen Aktivmasse wenigstens ein Molybdän und Vanadin enthaltendes Multimetalloxid ist, führt, daß der Acroleinumsatz bei einmaligem Durchgang ≥ 90 mol-% und die Selektivität der über beide Reaktionsstufen bilanzierten Acrylsäurebildung, bezogen auf umgesetztes Propen, ≥ 80 Mol-% beträgt, das dadurch gekennzeichnet ist, daß

a) die Belastung des ersten Festbettkatalysators mit dem im Reaktionsgasausgangsgemisch 1 enthaltenen Propen ≥ 160 Nl Propen/l Katalysatorschüttung · h beträgt,

b) die Belastung des zweiten Festbettkatalysators mit dem im Reaktionsgasausgangsgemisch 2 enthaltenen Acrolein ≥ 140 Nl Acrolein/l Katalysatorschüttung · h beträgt und

c) sowohl die Geometrie der Katalysatorformkörper des ersten Festbettkatalysators als auch die Geometrie der Katalysatorformkörper des zweiten Festbettkatalysators mit der Maßgabe ringförmig ist, daß

- der Ringaußendurchmesser 2 bis 11 mm,
- die Ringlänge 2 bis 11 mm und
- die Wandstärke des Ringes 1 bis 5 mm beträgt.

Erfindungsgemäß geeignete ringförmige Geometrien der Katalysatorformkörper der beiden Katalysatorfestbetten sind u.a. diejenigen, die in der EP-A 184790, der DE-A 3113179, der DE-A 3300044 und der EP-A 714700 beschrieben sind.

Ferner können die erfindungsgemäß geeigneten ringförmigen Katalysatorformkörper beider Reaktionsstufen sowohl Vollkatalysatoren (bestehen ausschließlich aus der katalytisch aktiven Multimetalloxidaktivmasse), Schalenkatalysatoren (ein ringförmiger Trägerkörper enthält auf seiner Außenfläche adsorptiv eine Schale der katalytisch aktiven Multimetalloxidmasse aufgebracht) als auch Trägerkatalysatoren (ein ringförmiger Trägerkörper enthält ein Absorbat der katalytisch aktiven Multimetalloxidmasse) sein.

Vorzugsweise werden beim erfindungsgemäßen verfahren in der ersten Reaktionsstufe ringförmige Vollkatalysatoren und in der zweiten Reaktionsstufe ringförmige Schalenkatalysatoren eingesetzt. Selbstverständlich können aber auch die Kombinationen "Schalenkatalysator/Vallkatalysator" oder "Vollkatalysatar/Vollkatalysator" oder "Schalenkatalysator/Schalenkatalysator" in den beiden aufeinanderfolgenden Raktionsstufen eingesetzt werden.

Im Fall von Schalenkatalysatoren werden erfindungsgemäß solche bevorzugt, deren Trägerringe eine Länge von 2 bis 10 mm (bzw. 3 bis 6 mm), einen Außendurchmesser von 2 bis 10 mm (bzw. 4 bis 8 mm) und eine Wanddicke von 1 bis 4 mm (bzw. 1 bis 2 mm) aufweisen. Ganz besonders bevorzugt besitzen die Trägerringe die Geometrie 7 mm x 3 mm x 4 mm (Außendurchmesser x Länge x Innendurchmesser). Die Dicke der auf den ringförmigen Trägerkörper als Schale aufgebrachten katalytisch aktiven Oxidmasse liegt normalerweise ganz generell bei 10 bis 1000 µm. Bevorzugt sind 50 bis 500 µm, besonders bevorzugt 100 bis 500 µm und ganz besonders bevorzugt 150 bis 250 µm.

Das vorstehend für die bevorzugte Geometrie der Trägerringe von erfindungsgemäß geeigneten Schalenkatalysatoren Gesagte gilt in gleicher Weise auch für die erfindungsgemäß geeigneten Trägerkatalysatoren.

Im Fall von erfindungsgemäß geeigneten Vollkatalysatorringen kommen insbesondere jene in Betracht, für die gilt, daß der Innendurchmesser das 0,1- bis 0,7-fache des Außendurchmessers und die Länge das 0,5- bis 2-fache des Außendurchmessers beträgt.

Günstige erfindungsgemäß verwendbare Vollkatalysatorringe haben einen Außendurchmesser von 2 bis 10 mm (bzw. 3 bis 7 mm), einen Ringinnendurchmesser von wenigstens 1,0 mm, eine Wanddicke von 1 bis 2 mm (bzw. höchstens 1,5 mm) und eine Länge von 2 bis 10 mm (bzw. 3 bis 6 mm). Häufig wird bei erfindungsgemäß geeigneten Vollkatalysatorringen der Außendurchmesser 4 bis 5 mm, der Innendurchmesser 1,5 bis 2,5 mm, die Wanddicke 1,0 bis 1,5 mm und die Länge 3 bis 6 mm betragen.

D.h., erfindungsgemäß geeignete Hohlzylindervollkatalysatorgeometrien sind (jeweils Außendurchmesser x Höhe x Innendurchmesser) die Geometrien 5 mm x 3 mm x 2 mm, 5 mm x 2 mm x 2 mm, 5 mm x 3 mm x 3 mm, 6 mm x 3 mm x 3 mm oder 7 mm x 3 mm x 4 mm.

Als Festbettkatalysatoren 1 kommen für das erfindungsgemäße Verfahren alle diejenigen in Betracht, deren Aktivmasse wenigstens ein Mo, Bi und Fe enthaltendes Multimetalloxid ist.

D.h., prinzipiell können alle diejenigen Multimetalloxide, die in den Schriften DE-C 3338380, DE-A 19902562, EP-A 15565,

DE-C 2380765, EP-A 807465, EP-A 279374, DE-A 3300044,

EP-A 575897, US-A 4438217, DE-A 19855913, WO 98/24746,

DE-A 19746210 (diejenigen der allgemeinen Formel II),

JP-A 91/294239, EP-A 293224 und EP-A 700714 offenbart werden, als Aktivmassen für Festbettkatalysatoren 1 verwendet werden. Dies gilt insbesondere für die beispielhaften Ausführungsformen in diesen Schriften, unter denen jene der EP-A 15565, der

EP-A 575897, der DE-A 19746210 und der DE-A 19855913 besonders bevorzugt werden. Besonders hervorzuheben sind in diesem Zusammenhang die Multimetalloxidaktivmasse gemäß Beispiel 1c aus der EP-A 15565 sowie eine in entsprechender Weise herzustellende Aktivmasse, die jedoch die Zusammensetzung $Mo_{12}Ni_{6,5}Zn_2Fe_2Bi_1$ $P_{0,0065}K_{0,06}O_x \cdot 10SiO_2$ aufweist.

Als erfindungsgemäß geeignete Festbettkatalysatoren sind hervorzuheben das Beispiel mit der laufenden Nr. 3 aus der DE-A 19855913 (Stöchiometrie: $MO_{12}CO_7Fe_3Bi_{0,6}K_{0,08}Si_{1,6}O_x$) als Hohlzylinder (Ring)vollkatalysator der Geometrie 5 mm x 3 mm x 2 mm (Außendurchmesser x Länge x Innendurchmesser) sowie der Multimetalloxid II-Vollkatalysator gemäß Beispiel 1 der DE-A 19746210. Ferner wären die Multimetalloxid-Ringvollkatalysatoren der US-A 4438217 zu nennen. Letzteres gilt insbesondere dann, wenn diese Hohlzylinder eine Geometrie 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Länge x Innendurchmesser) aufweisen.

Eine Vielzahl der für Festbettkatalysatoren 1 geeigneten Multimetalloxidaktivmassen läßt sich unter der allgemeinen Formel I

$$Mo_{12}Bi_aFe_bX^1_cX^2_dX^3_eX^4_fO_n \tag{I},$$

in der die Variablen nachfolgende Bedeutung aufweisen:

$X^1 =$ Nickel und/oder Kobalt,
$X^2 =$ Thallium, ein Alkalimetall und/oder ein Erdalkalimetall,
$X^3 =$ Zink, Phosphor, Arsen, Bor, Antimon, Zinn, Cer, Blei und/ oder Wolfram,
$X^4 =$ Silicium, Aluminium, Titan und/oder Zirkonium,

$a =$ 0,5 bis 5,
$b =$ 0,01 bis 5, vorzugsweise 2 bis 4,
$c =$ 0 bis 10, vorzugsweise 3 bis 10,
$d =$ 0 bis 2, vorzugsweise 0,02 bis 2,
$e =$ 0 bis 8, vorzugsweise 0 bis 5,
$f =$ 0 bis 10 und
$n =$ eine Zahl, die durch die Wertigkeit und Häufigkeit der von Sauerstoff verschiedenen Elemente in I bestimmt wird,

subsummieren.

Sie sind in an sich bekannter Weise erhältlich (siehe z.B. die DE-A 4023239) und werden erfindungsgemäß z.B. entweder in Substanz zu Ringen geformt oder in Gestalt von ringförmigen Schalenkatalysatoren, d.h., mit der Aktivmasse beschichteten ringförmig vorgeformten, inerten Trägerkörpern, eingesetzt.

Prinzipiell können für die Festbettkatalysatoren 1 geeignete Aktivmassen, insbesondere solche der allgemeinen Formel I, in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, Ammonium-Salze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu vollständig gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wäßrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Die für erfindungsgemäße Festbettkatalysatoren 1 geeigneten Multimetalloxidmassen, insbesondere jene der allgemeinen Formel I, werden für das erfindungsgemäße Verfahren zu ringförmiger Katalysatorgeometrie geformt eingesetzt, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Extrudieren) ringförmige Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können.

Selbstverständlich kann die Formgebung der pulverförmigen Aktivmasse oder ihrer pulverförmigen, noch nicht und/oder partiell calcinierten, Vorläufermasse auch durch Aufbringen auf ringförmig vorgeformte inerte Katalysatorträger erfolgen. Die Beschichtung der ringförmigen Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der ringförmigen Trägerkörper die aufzubringende Pulvermasse bzw. der Trägerkörper befeuchtet und nach dem Aufbringen, z.B. mittels heißer Luft, wieder getrocknet. Die Schichtdicke der auf den ringförmigen Trägerkörper aufgebrachten Pulvermasse wird zweckmäßigerweise im Bereich 10 bis 1000 μm, bevorzugt im Bereich 50 bis 500 μm und besonders bevorzugt im Bereich 150 bis 250 μm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Trägerkörper mit deutlich ausgebildeter Oberflächenrauhigkeit werden bevorzugt. Geeignet ist die Verwendung von im wesentlichen unporösen, oberflächenrauhen, ringförmigen Trägern aus Steatit. Zweckmäßig ist die Verwendung von ringförmigen Zylindern als Trägerkörper, deren Länge 2 bis 10 mm und deren Außendurchmesser 4 bis 10 mm beträgt. Die Wanddicke liegt üblicherweise bei 1 bis 4 mm. Erfindungsgemäß bevorzugt zu verwendende ringförmige Trägerkörper besitzen eine Länge von 3 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepaßt (vgl. EP-A 714 700).

Alternativ kann zum Zweck der Formgebung der ringförmige Trägerkörper auch mit einer die Ausgangsverbindungen der elementaren Konstituenten der relevanten Multimetalloxidaktivmasse enthaltenden Lösung und/oder Suspension getränkt, getrocknet und abschließend, wie beschrieben, unter Erhalt von Trägerkatalysatoren, calciniert werden.

Günstige für Festbettkatalysatoren 1 erfindungsgemäß zu verwendende Multimetalloxidaktivmassen sind ferner Massen der allgemeinen Formel II

$$[Y^1_{a'}, Y^2_{b'}O_{x'}]_p [Y^3_{c'}Y^4_{d'}Y^5_{e'}, Y^6_{f'}, Y^7_{g'}, Y^2_{h'}, O_{y'}]_q \qquad \text{(II)},$$

in der die Variablen folgende Bedeutung haben:

$Y^1$ =   Wismut, Tellur, Antimon, Zinn und/oder Kupfer,

$Y^2$ =   Molybdän und/oder Wolfram,

$Y^3$ =   ein Alkalimetall, Thallium und/oder Samarium,

$Y^4$ =   ein Erdalkalimetall, Nickel, Kobalt, Kupfer, Mangan, Zink, Zinn, Cadmium und/oder Quecksilber,

$Y^5$ =   Eisen, Chrom, Cer und/oder Vanadium,

$Y^6$ =   Phosphor, Arsen, Bor und/oder Antimon,

$Y^7$ =   ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

$a'$ =   0,01 bis 8,

$b'$ =   0,1 bis 30,

$c'$ =   0 bis 4,

$d'$ =   0 bis 20,

$e'$ =   0 bis 20,

f' =       0 bis 6,

g' =       0 bis 15,

h' =       8 bis 16,

x',y'=     Zahlen, die durch die Wertigkeit und Häufigkeit der von Sauerstoff verschiedenen Elemente in II bestimmt werden und

p,q =      Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}, Y^2_{b'}, O_{x'}$, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 μm, häufig 10 nm bis 500 nm oder 1 μm bis 50 bzw. 25 μm, beträgt.

Besonders vorteilhafte erfindungsgemäße Multimetalloxidmassen II sind solche, in denen $Y^1$ Wismut ist.

Unter diesen werden wiederum jene bevorzugt, die der allgemeinen Formel III,

$$[Bi_{a''} Z^2_{b''} O_{x''}]_{p''} \ [Z^2_{12} Z^3_{c''} Z^4_{d''} Fe_{e''} Z^5_{f''} Z^6_{g''} Z^7_{h''} O_{y''}]_{q''} \tag{III},$$

in der die Varianten folgende Bedeutung haben:

$Z^2$ =     Molybdän und/oder Wolfram,

$Z^3$ =     Nickel und/oder Kobalt,

$Z^4$ =     Thallium, ein Alkalimetall und/oder ein Erdalkalimetall,

$Z^5$ =     Phosphor, Arsen, Bor, Antimon, Zinn, Cer und/oder Blei,

$Z^6$ =     Silicium, Aluminium, Titan und/oder Zirkonium,

$Z^7$ =     Kupfer, Silber und/oder Gold,

a" =       0,1 bis 1,

b" =       0,2 bis 2,

c" =       3 bis 10,

d" =       0,02 bis 2,

e" =       0,01 bis 5, vorzugsweise 0,1 bis 3,

f " =      0 bis 5,

g" =       0 bis 10,

h" =       0 bis 1,

x",y"=     Zahlen, die durch die Wertigkeit und Häufigkeit der von Sauerstoff verschiedenen Element in III bestimmt werden,

p",q"=     Zahlen, deren Verhältnis p"/q" 0,1 bis 5, vorzugsweise 0,5 bis 2 beträgt,

entsprechen, wobei diejenigen Massen III ganz besonders bevorzugt werden, in denen $Z^2_{b''}$ = (Wolfram)$_{b''}$ und $Z^2_{12}$ = (Molybdän)$_{12}$ ist.

Ferner ist es von Vorteil, wenn wenigstens 25 mol-% (bevorzugt wenigstens 50 mol-% und besonders bevorzugt wenigstens 100 mol-%) des gesamten Anteils ($y^1_a$, $y^2_b$, $O_x$]$_p$ ([$Bi_{a''}Z^2_{b''}O_{x''}]_{p''}$) der als Festbettkatalysatoren 1 erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $y^1_a, y^2_b, O_x$, ($Bi_{a''}Z^2_{b''}O_{x''}$) vorliegen, deren Größtdurchmesser im Bereich 1 nm bis 100 μm liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z.B. in der EP-A 575897 sowie in der DE-A 19855913 beschrieben.

Üblicherweise erfolgt die Durchführung der ersten Reaktionsstufe des erfindungsgemäßen Verfahrens in einem mit den ringförmigen Katalysatoren beschickten Rohrbündelreaktor wie er z.B. in der EP-A 700714 beschrieben ist.

D.h., in einfachster Weise befindet sich der erfindungsgemäß zu verwendende Festbettkatalysator 1 in den Metallrohren eines Rohrbündelreaktors und um die Metallrohre wird ein Temperaturmedium (Einzonenfahrweise), in der Regel eine Salzschmelze, geführt. Salzschmelze und Reaktionsgasgemisch können dabei im einfachen Gleich- oder Gegenstrom geführt werden. Die Salzschmelze (das Temperiermedium) kann aber auch über den Reaktor betrachtet mäanderförmig um die Rohrbündel geführt werden, so daß lediglich über den gesamten Reaktor betrachtet ein Gleich- oder Gegenstrom zur Strömungsrichtung des Reaktionsgasgemisches besteht. Die Strömungsgeschwindigkeit des Temperiermediums (Wärmeaustauschmittels) wird dabei üblicherweise so bemessen, daß der Temperaturanstieg (bedingt durch die Exothermie der Reaktion) des Wärmeaustauschmittels von der Eintrittsstelle in den Reaktor bis zur Austrittstelle aus dem Reaktor ≥ 0 bis 10°C, häufig ≥ 2 bis 8°C, oft ≥ 3 bis 6°C beträgt. Die Eintrittstemperatur des Wärmeaustauschmittels in den Rohrbündelreaktor beträgt in der Regel 310 bis 360°C, häufig 320 bis 340°C.

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium Quecksilber sowie Legierungen verschiedener Metalle.

Zweckmäßigerweise wird das Reaktionsgasausgangsgemisch 1 der Katalysatorbeschickung 1 auf die Reaktionstemperatur vorerwärmt zugeführt. Diese beträgt bei der ebenda beschriebenen erfindungsgemäßen Variante der ersten Reaktionsstufe häufig 310 bis 360°C, vielfach 320 bis 340°C.

In anwendungstechnisch zweckmäßiger Weise erfolgt die Durchführung der ersten Reaktionsstufe des erfindungsgemäßen Verfahrens in einem Zweizonenrohrbündelreaktor. Eine bevorzugte variante eines erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903218 offenbarten Zweizonenrohrbündelreaktoren sind für eine Durchführung.der ersten Reaktionsstufe des erfindungsgemäßen Verfahrens geeignet.

D.h., in einfachster Weise befindet sich der erfindungsgemäß zu verwendende Festbettkatalysator 1 in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Reaktionszone. D.h., in einfachster Weise umströmt ein Salzbad A denjenigen Abschnitt der Rohre (die Reaktionszone A), in welchem sich die oxidative Umsetzung des Propens (beim einfachen Durchgang) bis zum Erreichen eines Umsatzes im Bereich von 40 bis 80 mol-% vollzieht und ein Salzbad B umströmt den Abschnitt der Rohre (die Reaktionszone B), in welchem sich die oxidative Anschlußumsetzung des Propens (beim einfachen Durchgang) bis zum Erreichen eines Umsatzwertes von wenigstens 90 mol-% vollzieht (bei Bedarf können sich an die erfindungsgemäß anzuwendenden Reaktionszonen A,B weitere Reaktionszonen anschließen, die auf individuellen Temperaturen gehalten werden).

Anwendungstechnisch zweckmäßig umfaßt die erste Reaktionsstufe des erfindungsgemäßen Verfahrens keine weiteren Reaktionszonen. D.h., das Salzbad B umströmt zweckmäßig den Abschnitt der Rohre, in welchem sich die oxidative Anschlußumsetzung des Propens (beim einfachen Durchgang) bis zu einem Umsatzwert ≥ 92 mol-% oder ≥ 94 mol-% oder mehr vollzieht.

Üblicherweise liegt der Beginn der Reaktionszone B hinter dem Heißpunktmaximum der Reaktionszone A. Das Heißpunktmaximum der Reaktionszone B liegt normalerweise unterhalb der Heißpunktmaximaltemperatur der Reaktionszone A.

Die beiden Salzbäder A,B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Reaktionszone A eine Gleichströmung und in der Reaktionszone B eine Gegenströmung (oder umgekehrt) angewandt werden.

Selbstverständlich kann man in allen vorgenannten Fallkonstellationen innerhalb der jeweiligen Reaktionszone der, relativ zu den Reaktionsrohren, erfolgenden Parallelströmung der Salzschmelze noch eine Querströmung überlagern, so daß die einzelne Reaktionszone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird das Reaktionsgasausgangsgemisch 1 der Katalysatorbeschickung 1 auf die Reaktionstemperatur vorerwärmt zugeführt.

Üblicherweise sind in den vorgenannten Rohrbündelreaktoren (dies gilt auch für die Einzonenrohrbündelreaktoren) die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Anwendungstechnisch zweckmäßig beläuf t sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, daß der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium Quecksilber sowie Legierungen verschiedener Metalle.

In der Regel wird bei allen vorstehend erwähnten Konstellationen der Stromführung in den Zweizonenrohrbündelreaktoren die Fließgeschwindigkeit innerhalb der beiden erforderlichen Wärmeaustauschmittelkreisläufen so gewählt, daß die Temperatur des Wärmeaustauschmittels von der Eintrittsstelle in die Reaktionszone bis zur Austrittsstelle aus der Reaktionszone (bedingt durch die Exothermie der Reaktion) um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in die Reaktionszone A beträgt erfindungsgemäß normalerweise 310 bis 340°C. Die Eintrittstemperatur des Wärmeaustauschmittels in die Reaktionszone B beträgt erfindungsgemäß normalerweise einerseits 315 bis 380°C und liegt andererseits gleichzeitig wenigstens 5°C oberhalb der Eintrittstemperatur des in die Reaktionszone A eintretenden Wärmeaustauschmittels.

Bevorzugt liegt die Eintrittstemperatur des Wärmeaustauschmittels in die Reaktionszone B wenigstens 10°C oberhalb der Eintrittstemperatur des in die Reaktionszone A eintretenden Wärmeaustauschmittels. Die Differenz zwischen den Eintrittstemperaturen in die Reaktionszone A bzw. B kann erfindungsgemäß somit bis zu 20°C, bis zu 25°C, bis zu 30°C, bis zu 40°C, bis zu 45°C oder bis zu 50°C betragen. Normalerweise wird die vorgenannte Temperaturdifferenz aber nicht mehr als 50°C betragen. Je höher die Propenbelastung der Katalysatorschüttung. 1 beim erfindungsgemäßen Verfahren gewählt wird, um so größer sollte die Differenz zwischen der Eintrittstemperatur des Wärmeaustauschmittels in die Reaktionszone A und der Eintrittstemperatur des Wärmeaustauschmittels in die Reaktionszone B sein.

Mit Vorteil beträgt die Eintrittstemperatur des Wärmeaustauschmittels in die Reaktionszone B erfindungsgemäß 330 bis 370°C und besonders vorteilhaft 340 bis 370°C.

Selbstverständlich können beim erfindungsgemäßen Verfahren die beiden Reaktionszonen A, B auch in räumlich voneinander getrennten Rohrbündelreaktoren realisiert sein. Bei Bedarf kann zwischen den beiden Reaktionszonen A,B auch ein Wärmetauscher angebracht werden. Selbstredend können die beiden Reaktionszonen A, B auch als Wirbelbett gestaltet werden.

Ferner können beim erfindungsgemäßen Verfahren (sowohl bei der Einzonen- als auch bei der Zweizonenvariante) auch Katalysatorschüttungen 1 verwendet werden, deren volumenspezifische Aktivität in Strömungsrichtung des Reaktionsgasausgangsgemisches 1 kontinuierlich, abrupt oder stufenförmig zunimmt (dies kann z.B. wie in der WO 98/24746 oder wie in der JP-A 91/294239 beschrieben oder auch durch Verdünnung mit Inertmaterial bewirkt werden). Ebenso können neben Stickstoff, Wasserdampf und/oder Kohlenoxiden die in der EP-A 293224 und in der EP-B 257565 empfohlenen inerten Verdünnungsgase (z.B. nur Propan oder nur Methan etc.) eingesetzt werden. Letzteres bei Bedarf auch kombiniert mit einer in Strömungsrichtung des Reaktionsgasgemisches zunehmenden volumenspezifischen Aktivität der Katalysatorschüttung.

Es sei an dieser Stelle auch noch einmal darauf hingewiesen, daß für eine Durchführung der Reaktionsstufe 1 des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Reaktionszone B eine Teilmenge an die Reaktionszone A abzuführen, um gegebenenfalls ein Anwärmen eines kalten Reaktionsgasausgangsgemisches oder eines kalten Kreisgases zu bewirken. Ferner kann die Rohrbündelcharakteristik innerhalb einer individuellen Reaktionszone wie in der EP-A 382098 beschrieben gestaltet werden.

Erfindungsgemäß hat es sich als zweckmäßig erwiesen, das die erste Reaktionsstufe verlassende Produktgasgemisch vor dem Eintritt in die zweite Reaktionsstufe abzukühlen, um so eine Nachvollverbrennung von Teilen des in der ersten Reaktionsstufe gebildeten Acroleins zu unterdrücken. Üblicherweise wird dazu zwischen die beiden Reaktionsstufen ein Nachkühler geschaltet. Dies kann im einfachsten Fall ein indirekter Rohrbündelwärmeüberträger sein. Das Produktgasgemisch wird dabei in der Regel durch die Rohre geführt und um die Rohre wird ein Wärmetauschermedium geführt, dessen Art der für die Rohrbündelreaktoren empfohlenen Wärmetauschermedien entsprechen kann. Mit Vorteil ist das Rohrinnere mit inerten Füllkörpern (z.B. Spiralen aus Edelstahl, Ringe aus Steatit, Kugeln aus Steatit etc.) gefüllt. Selbige verbessern den Wärmeaustausch und fangen gegebenenf alls aus der Festbettkatalysatorschüttung der ersten Reaktionsstufe sublimierendes Molybdäntrioxid vor einem Eintritt desselben in die zweite Reaktionsstufe ab. Es ist von Vorteil, wenn der Nachkühler aus mit Zinksilicatfarbe beschichtetem rostfreiem Stahl gefertigt ist.

In der Regel wird der auf den einfachen Durchgang bezogene Propenumsatz beim erfindungsgemäßen Verfahren in der ersten Reaktionsstufe ≥ 92 mol-% oder ≥ 94 mol-% betragen. Die in der ersten Reaktionsstufe bei einfachem Durchgang resultierende Selektivität der Acroleinbildung sowie der Acrylsäurenebenproduktbildung wird dabei regelmäßig ≥ 92 mol-% oder ≥ 94 mol-%, häufig ≥ 95 mol-% oder ≥ 96 mol-% bzw. ≥ 97 mol-% betragen.

Das erfindungsgemäße Verfahren eignet sich für Propenbelastungen der Katalysatorschüttung 1 von ≥ 165 Nl/l·h oder von ≥ 170 Nl/l·h bzw. ≥ 175 Nl/l·h oder ≥ 180 Nl/l·h, aber auch für Propenbelastungen der Katalysatorschüttung 1 von ≥ 185 Nl/l·h oder ≥ 190 Nl/l·h bzw. ≥ 200 Nl/l·h oder ≥ 210 Nl/l·h sowie für Belastungswerte von ≥ 220 Nl/l·h oder ≥ 230 Nl/l·h bzw. ≥ 240 Nl/l·h oder ≥ 250 Nl/l·h.

Dabei kann das für das Reaktionsgasausgangsgemisch 1 erfindungsgemäß zu verwendende Inertgas zu $\geq$ 20 Vol.-%, oder zu $\geq$ 30 Vol.-%, oder zu $\geq$ 40 Vol.-%, oder zu $\geq$ 50 Vol.-%, oder zu $\geq$ 60 Vol.-%, oder zu $\geq$ 70 Vol.-%, oder zu $\geq$ 80 Vol.-%, oder zu $\geq$ 90 Vol.-%, oder zu $\geq$ 95 Vol.-% aus molekularem Stickstoff bestehen.

Bei Propenbelastungen der Katalysatorschüttung 1 oberhalb von 250 Nl/l·h wird für das erfindungsgemäße Verfahren jedoch die Mitverwendung von inerten (inerte Verdünnungsgase sollen hier generell solche sein, die sich beim einmaligen Durchgang durch die jeweilige Reaktionsstufe zu weniger als 5 %, bevorzugt zu weniger als 2 % umsetzen) Verdünnungsgasen wie Propan, Ethan, Methan, Pentan, Butan, $CO_2$, CO, Wasserdampf und/oder Edelgasen für das Reaktionsgasausgangsgemisch 1 empfohlen. Selbstverständlich können diese Gase und ihre Gemische aber auch bereits bei geringeren erf indungsgemäßen Propenbelastungen der Katalysatorschüttung 1 im Reaktionsgasausgangsgemisch 1 mitverwendet oder als alleinige Verdünnungsgase verwendet werden. Es überrascht, daß das erfindungsgemäße Verfahren generell auch bei einer homogenen, d.h. chemisch einheitlichen, Katalysatorschüttung 1 mit guten Selektivitäten durchgeführt werden kann.

Mit zunehmender Propenbelastung ist die beschriebene Zweizonenfahrweise gegenüber der beschriebenen Einzonenfahrweise in der ersten Reaktionsstufe bevorzugt.

Normalerweise wird beim erfindungsgemäßen Verfahren die Propenbelastung des ersten Festbettkatalysators den Wert 600 Nl/l·h nicht überschreiten. In typischer Weise liegen die Propenbelastungen des ersten Festbettkatalysators beim erfindungsgemäßen Verfahren bei Werten $\leq$ 300 Nl/l·h, häufig bei Werten $\leq$ 250 Nl/l·h.

Der Arbeitsdruck kann beim erfindungsgemäßen Verfahren in der ersten Reaktionsstufe sowohl unterhalb von Normaldruck (z.B. bis zu 0,5 bar) als auch oberhalb von Normaldruck liegen. Typischerweise wird der Arbeitsdruck in der ersten Reaktionsstufe bei Werten von 1 bis 5 bar, häufig 1,5 bis 3,5 bar liegen. Normalerweise wird der Reaktionsdruck in der ersten Reaktionsstufe 100 bar nicht überschreiten.

Das molare Verhältnis von $O_2 : C_3H_6$ im Reaktionsgasausgangsgemisch 1 muß erfindungsgemäß $\geq$ 1 betragen. Üblicherweise wird dieses Verhältnis bei Werten $\leq$ 3 liegen. Häufig beträgt das molare Verhältnis von $O_2 : C_3H_6$ im Reaktionsgasausgangsgemisch 1 erfindungsgemäß $\geq$ 1,5 und $\leq$ 2,0.

Als Quelle für den in der ersten Reaktionsstufe erforderlichen molekularen Sauerstoff kommt sowohl Luft, als auch an molekularem Stickstoff entreicherte Luft(z.B. $\geq$ 90 Vol.-% $O_2$, $\leq$ 10 Vol-% $N_2$) in Betracht.

Der Propenanteil im Reaktionsgasausgangsgemisch 1 kann erfindungsgemäß z.B. bei Werten von 4 bis 15 Vol.-%, häufig bei 5 bis 12 Vol-% bzw. 5 bis 8 Vol.-% liegen (jeweils bezogen auf das Gesamtvolumen).

Häufig wird man das erfindungsgemäße Verfahren bei einem Propen:Sauerstoff:indifferente Gase (einschließlich Wasserdampf) Volumenverhältnis im Reaktionsgasausgangsgemisch 1 von 1: (1,0 bis 3,0):(5 bis 25), vorzugsweise 1:(1,5 bis 2,3):(10 bis 15) durchführen.

In der Regel enthält das Reaktionsgasausgangsgemisch 1 neben den genannten Bestandteilen im wesentlichen keine weiteren Komponenten.

Anwendungstechnisch zweckmäßig wird das Produktgasgemisch der ersten Reaktionsstufe im bereits erwähnten Nachkühler auf eine Temperatur von 210 bis 290°C, häufig 220 bis 260°C oder 225 bis 245°C abgekühlt. Dabei kann die Abkühlung des Produktgasgemisches der ersten Reaktionsstufe durchaus auf Temperaturen erfolgen, die unterhalb der Temperatur der zweiten Reaktionsstufe liegen. Die beschriebene Nachkühlung ist jedoch keineswegs zwingend und kann insbesondere dann in aller Regel entfallen, wenn der Weg des Produktgasgemisches von der ersten Reaktionsstufe in die zweite Reaktionsstufe kurz gehalten wird. Üblicherweise wird das erfindungsgemäße Verfahren ferner so verwirklicht, daß man den Sauerstoffbedarf in der zweiten Reaktionsstufe nicht bereits durch einen entsprechend hohen Sauerstoffgehalt des Reaktionsgasausgangsgemisches 1 deckt, sondern den benötigten Sauerstoff im Bereich zwischen erster und zweiter Reaktionsstufe zugibt. Dies kann vor, während nach und/oder zur Nachkühlung erfolgen. Als Quelle für den in der zweiten Reaktionsstufe erforderlichen molekularen Sauerstoff kommen sowohl reiner Sauerstoff als auch Gemische aus Sauerstoff und Inertgas, z.B. Luft oder an molekularem Stickstoff entreicherte Luft (z.B.$\geq$ 90 Vol-% $O_2$, $\leq$ 10 Vol-% $N_2$) in Betracht. Die Zugabe der Sauerstoffquelle erfolgt regelmäßig in auf den Reaktionsdruck komprimierter Form.

Der Acroleinanteil im so erzeugten Reaktionsgasausgangsgemisch 2 kann erfindungsgemäß z.B. bei Werten von 3 bis 15 Vol.-%, häufig bei 4 bis 10 Vol-% bzw. 5 bis 8 Vol-% liegen (jeweils bezogen auf das Gesamtvolumen).

Erfindungsgemäß muß das molare Verhältnis von $O_2$:Acrolein im Reaktionsgasausgangsgemisch 2 $\geq$ 0,5 bzw. 1 betragen. Üblicherweise wird dieses Verhältnis bei Werten $\leq$ 3 liegen. Häufig wird das molare Verhältnis von $O_2$:Acrolein im Reaktionsgasausgangs-gemisch 2 erfindungsgemäß 1 bis 2 bzw. 1 bis 1,5 betragen. Häufig wird man das erfindungsgemäße Verfahren mit einem im Reaktionsgasausgangsgemisch 2 vorliegenden Acrolein:Sauerstoff: Wasserdampf:Inertgas-Volumenverhältnis (N1) von 1: (0,5 bzw. 1 bis 3): (0 bis 20) : (3 bis 30) , vorzugsweise von 1: (1 bis 3) : (0, 5 bis 10):(7 bis 10) ausführen.

Der Arbeitsdruck kann in der zweiten Reaktionsstufe sowohl unterhalb von Normaldruck (z.B. bis zu 0,5 bar) als auch oberhalb von Normaldruck liegen. Typischerweise wird der Arbeitsdruck in der zweiten Reaktionsstufe erfindungsgemäß bei Werten von 1 bis 5 · bar, häufig 1 bis 3 bar liegen. Normalerweise wird der Reaktionsdruck in der zweiten Reaktionsstufe 100 bar nicht überschreiten.

Ebenso wie die erste Reaktionsstufe kann die zweite Reaktionsstufe des erfindungsgemäßen Verfahrens in einfacher Weise in einem mit den ringförmigen Katalysatoren beschickten Rohrbündelreaktor, wie er z.B. in der EP-A 700893 beschrieben ist, durchgeführt werden.

D.h., in einfachster Weise befindet sich der erfindungsgemäß zu verwendende Festbettkatalysator 2 in den Metallrohren eines Rohrbündelreaktors und um die Metallrohre wird ein Temperiermedium (Einzonenfahrweise), in der Regel eine Salzschmelze geführt. Salzschmelze und Reaktionsgasgemisch können dabei im einfachen Gleich- oder Gegenstrom geführt werden. Das Temperiermedium kann aber auch über den Reaktor betrachtet mäanderförmig um die Rohrbündel geführt werden, so daß lediglich über den gesamten Reaktor betrachtet ein Gleich- oder Gegenstrom zur Strömungsrichtung des Reaktionsgasgemisches besteht. Der Volumenstrom des Temperiermediums (Wärmeaustauschmittels) wird dabei üblicherweise so bemessen, daß der Temperaturanstieg (bedingt durch die Exothermie der Reaktion) des Wärmeaustauschmittels von der Eintrittstelle in den Reaktor bis zur Austrittstelle aus dem Reaktor $\geq 0$ bis 10°C, häufig $\geq 2$ bis 8°C, oft $\geq 3$ bis 6°C beträgt. Die Eintrittstemperatur des Wärmeaustauschmittels in den Rohrbündelreaktor beträgt in der Regel 230 bis 300°C, häufig 245 bis 285°C bzw. 245 bis 265°C. Als Wärmeaustauschmittel eignen sich dabei die gleichen fluiden Temperiermedien, wie sie bereits für die erste Reaktionsstufe beschrieben worden sind.

Zweckmäßigerweise wird das Reaktionsgasausgangsgemisch 2 der Katalysatorbeschickung 2 auf die Reaktionstemperatur vorerwärmt zugeführt. Diese beträgt bei der ebenda beschriebenen erfindungsgemäßen Variante der zweiten Reaktionsstufe häufig 230 bis 300°C, vielfach 245 bis 285°C bzw. 245 bis 265°C.

In der Regel wird eine Einzonenfahrweise der ersten Reaktionsstufe mit. einer Einzonenfahrweise der zweiten Reaktionsstufe kombiniert, wobei die relative Stromführung vom Reaktionsgasgemisch und Temperiermedium in beiden Stufen identisch gewählt wird.

Selbstverständlich kann aber auch die zweite Reaktionsstufe des erfindungsgemäßen Verfahrens in entsprechender Weise wie die erste Reaktionsstufe als zwei räumlich aufeinander folgende Reaktionszonen C, D realisiert werden, wobei die Temperatur der Reaktionszone C zweckmäßig 230 bis 270°C und die Temperatur der Reaktionszone D 250 bis 300°C beträgt und gleichzeitig wenigstens 10°C oberhalb der Temperatur der Reaktionszone C liegt.

Bevorzugt erstreckt sich die Reaktionszone C bis zu einem Acroleinumsatz von 65 bis 80 mol-%. Außerdem liegt die Temperatur der Reaktionszone C mit vorteil bei 245 bis 260°C. Die Temperatur der Reaktionszone D liegt vorzugsweise wenigstens 20°C oberhalb der Temperatur der Reaktionszone C und beträgt vorteilhaft 265 bis 285°C.

Je höher die Acroleinbelastung der Katalysatorschüttung 2 beim erfindungsgemäßen Verfahren gewählt wird, um so größer sollte die Differenz zwischen der Temperatur der Reaktionszone C und der Temperatur der Reaktionszone D gewählt werden. Normalerweise wird die vorgenannte Temperaturdifferenz beim erfindungsgemäßen Verfahren aber nicht mehr als 40°C betragen. D.h., die Differenz zwischen der Temperatur der Reaktionszone C und der Temperatur der Reaktionszone D kann erfindungsgemäß bis zu 15°C, bis zu 25°C, bis zu 30°C, bis zu 35°C oder bis zu 40°C betragen.

Generell kann beim erfindungsgemäßen Verfahren der auf den einfachen Durchgang der zweiten Reaktionsstufe bezogene Acroleinumsatz beim erfindungsgemäßen Verfahren $\geq 92$ mol-%, oder $\geq 94$ mol-%, oder $\geq 96$ mol-%, oder $\geq 98$ mol-% und häufig sogar $\geq 99$ mol-% betragen. Die Selektivität der Acrylsäurebildung, bezogen auf umgesetztes Acrolein, kann dabei regelmäßig $\geq 92$ mol-%, bzw.$\geq 94$ mol-%, häufig $\geq 95$ mol-% oder $\geq 96$ mol-% bzw. $\geq 97$ mol-% betragen.

Das erfindungsgemäße Verfahren eignet sich für Acroleinbelastungen der Katalysatorschüttung 2 von $\geq 140$ Nl/l·h bzw. $\geq 150$ Nl/l·h oder von $\geq 160$ Nl/l·h bzw. $\geq 170$ Nl/l·h oder $\geq 175$ Nl/l·h bzw. $\geq 180$ Nl/l·h, aber auch bei Acroleinbelastungen der Katalysatorschüttung 2 von $\geq 185$ Nl/l·h oder von $\geq 190$ Nl/l·h bzw. $\geq 200$ Nl/l·h oder $\geq 210$ Nl/l·h sowie bei Belastungswerten $\geq 220$ Nl/l·h oder $\geq 230$ Nl/l·h bzw. 240 Nl/l·h oder $\geq 250$ Nl/l·h.

Dabei kann das in der zweiten Reaktionsstufe erfindungsgemäß mitzuverwendende Inertgas zu $\geq 30$ Vol-%, oder zu $\geq 40$ Vo1-%, oder zu $\geq 50$ Vol-%, oder zu $\geq 60$ Vol-%, oder zu $\geq 70$ Vol-%, oder zu $\geq 80$ Vol-%, oder zu $\geq 90$ Vol-%, oder zu $\geq 95$ Vol-% aus molekularem Stickstoff bestehen.

In zweckmäßiger Weise wird das inerte Verdünnungsgas beim erfindungsgemäßen Verfahren in der zweiten Reaktionsstufe zu 5 bis 20 Gew.-% aus $H_2O$ (wird in der ersten Reaktionsstufe gebildet) und zu 70 bis 90 Vol-% aus $N_2$ bestehen.

Außer den in dieser Schrift genannten Bestandteilen enthält das Reaktionsgasausgangsgemisch 2 normalerweise im wesentlichen keine weiteren Komponenten.

Bei Acroleinbelastungen des zweiten Festbettkatalysators oberhalb von 250 Nl/l·h wird für das Reaktionsgasausgangsgemisch 2 die Mitverwendung von inerten Verdünnungsgasen wie Propan, Ethan, Methan, Butan, Pentan, $CO_2$, CO, Wasserdampf und/oder Edelgasen empfohlen. Selbstverständlich können diese Gase aber auch bereits bei geringeren Acroleinbelastungen mitverwendet werden. Generell kann das erfindungsgemäße Verfahren bei guten Selektivitäten mit einer homogenen, d.h., einer chemisch einheitlichen, Katalysatorschüttung 2 durchgeführt werden.

Normalerweise wird beim erfindungsgemäßen Verfahren die Acroleinbelastung des zweiten Festbettkatalysators den Wert von 600 Nl/l·h nicht überschreiten. In typischer Weise liegen die Acroleinbelastungen der Katalysatorschüttung 2 beim erfindungsgemäßen Verfahren ohne nennenswerten Verlust von Umsatz und Selektivität bei Werten ≤ 300 Nl/l·h, häufig bei Werten ≤ 250 Nl/l·h.

In der Regel wird beim erfindungsgemäßen Verfahren die Acroleinbelastung der zweiten Katalysatorschüttung etwa 10 Nl/l·h, häufig etwa 20 bzw. 25 Nl/l·h unterhalb der Propenbelastung der ersten Katalysatorschüttung liegen. Dies ist primär darauf zurückzuführen, daß in der ersten Reaktionsstufe sowohl Umsatz als auch Selektivität zu Acrolein in der Regel nicht 100 % erreichen. Ferner wird der Sauerstoffbedarf der zweiten Reaktionsstufe üblicherweise durch Luft gedeckt. Mit zunehmender Acroleinbelastung ist die beschriebene Zweizonenfahrweise gegenüber der ausgeführten Einzonenfahrweise in der zweiten Reaktionsstufe bevorzugt.

Beachtenswerterweise kann beim erfindungsgemäßen Verfahren die über beide Reäktionsstufen bilanzierte Selektivität der Acrylsäurebildung, bezogen auf umgesetztes Propen, selbst bei höchsten Propen- und Acroleinbelastungen in der Regel bei Werten ≥ 83 mol-%,häufig bei ≥ 85 mol-% oder ≥ 88 mol-%, oft bei ≥ 90 mol-% oder 93 mol-% liegen.

Als erfindungsgemäß zu verwendende ringförmige Festbettkatalysatoren 2 kommen für die gasphasenkatalytische Acroleinoxidation in der zweiten Reaktionsstufe alle diejenigen in Betracht, deren Aktivmasse wenigstens ein Mo und V enthaltendes Multimetalloxid ist.

Solchermaßen geeignete Multimetalloxidaktivmassen können beispielsweise der US-A 3 775.474, der US-A 3 954 855, der US-A 3 893 951 und der US-A 4 339 355 entnommen werden. Ferner eignen sich in besonderer Weise die Multimetalloxidaktivmassen der EP-A 427 508, der DE-A 2 909 671, der DE-C 31 51 805, der DE-AS 2 626 887, der DE-A 43 02 991, der EP-A 700 893, der EP-A 714 700 und der DE-A 19 73 6105. Besonders bevorzugt sind in diesem Zusammenhang die beispielhaften Ausführungsformen der EP-A 714 700 sowie der DE-A 19 73 6105.

Eine Vielzahl der für Festbettkatalysatoren 2 geeigneten Multimetalloxidaktivmassen läßt sich unter der allgemeinen Formel IV

$$Mo_{12}V_aX^1_bX^2_cX^3_dX^4_eX^5_fX^6_gO_n \qquad (IV),$$

in der die Variablen folgende Bedeutung haben:

$X^1 =$ W, Nb, Ta, Cr und/oder Ce,
$X^2 =$ Cu, Ni, Co, Fe, Mn und/oder Zn,
$X^3 =$ Sb und/oder Bi,
$X^4 =$ eines oder mehrere Alkalimetalle,
$X^5 =$ eines oder mehrere Erdalkalimetalle,
$X^6 =$ Si, Al, Ti und/oder Zr,
$a =$ 1 bis 6,
$b =$ 0,2 bis 4,
$c =$ 0,5 bis 18,
$d =$ 0 bis 40,
$e =$ 0 bis 2,
$f =$ 0 bis 4,
$g =$ 0 bis 40 und
$n =$ eine Zahl, die durch die Wertigkeit und Häufigkeit der von Sauerstoff verschiedenen Elemente in IV bestimmt wird,

subsummieren.

Bevorzugte Ausführungsformen innerhalb der aktiven Multimetalloxide IV sind jene, die von nachfolgenden Bedeutungen der Variablen der allgemeinen Formel IV erfaßt werden:

$X^1 =$ W, Nb, und/oder Cr,
$X^2 =$ Cu, Ni, Co, und/oder Fe,
$X^3 =$ Sb,
$X^4 =$ Na und/oder K,
$X^5 =$ Ca, Sr und/oder Ba,
$X^6 =$ Si, Al, und/oder Ti,
$a =$ 1,5 bis 5,
$b =$ 0,5 bis 2,
$c =$ 0,5 bis 3,

d = 0 bis 2,

e = 0 bis 0,2,

f = 0 bis 1 und

n = eine Zahl, die durch die wertigkeit und Häufigkeit der von Sauerstoff verschiedenen Elemente in IV bestimmt wird.

Ganz besonders bevorzugte Multimetalloxide IV sind jedoch jene der allgemeinen Formel V

$$MO_{12}V_{a'}Y^1_{b'}\,Y^2_{c'}Y^5_{f'}Y^6_{g'}O_{n'} \qquad\qquad (V)$$

mit

$Y^1$ = W und/oder Nb,

$Y^2$ = Cu und/oder Ni,

$Y^5$ = Ca und/oder Sr,

$Y^6$ = Si und/oder Al,

a' = 2 bis 4,

b' = 1 bis 1,5,

c' = 1 bis 3,

f' = 0 bis 0,5,

g' = 0 bis 8 und

n' = eine Zahl, die durch die Wertigkeit und Häufigkeit der von Sauerstoff verschiedenen Elementen in v bestimmt wird.

Die erfindungsgemäß geeigneten Multimetalloxidaktivmassen (IV) sind in sich bekannter, z.B. in der DE-A 4335973 oder in der EP-A 714700 offenbarter, Weise erhältlich.

Prinzipiell können erfindungsgemäß für Festbettkatalysatoren 2 geeignete Multimetalloxidaktivmassen, insbesondere solche der allgemeinen Formel IV, in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie $H_2$, $NH_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wäßrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Die für Festbettkatalysatoren 2 geeigneten Multimetalloxidmassen, insbesondere jene der allgemeinen Formel IV, werden für das erfindungsgemäße Verfahren zu ringförmigen Katalysatorgeometrien geformt eingesetzt, wobei die Formgebung in völlig entsprechender Weise wie bei den Festbettkatalysatoren 1 vor oder nach der abschließenden Calcination erfolgen kann. Beispielsweise können ganz analog aus der Pulverform der Aktivmasse oder ihrer uncalcinierten Vorläufermasse durch Verdichten zur gewünschten Katalysatorgeometrie (z.B. durch Extrudieren) ringförmige Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind, wie bereits genannt, Hohlzylinder mit einem Außendurchmesser und einer Länge von 2 bis 10 mm. Dabei ist eine Wandstärke von 1 bis 3 mm zweckmäßig.

Selbstverständlich kann die Formgebung der pulverförmigen Aktivmasse oder ihrer pulverförmigen, noch nicht calcinierten, Vorläufermasse auch durch Aufbringen auf ringförmig vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper mit deutlich ausgebildeter Oberflächenrauhigkeit werden bevorzugt. Geeignet ist die Verwendung von Hohlzylindern als Trägerkörper, deren Länge 2 bis 10 mm und deren Außendurchmesser 4 bis 10 mm beträgt. Die wanddicke liegt darüber hinaus üblicherweise bei 1 bis 4 mm. Erfindungsgemäß bevorzugt zu verwendende ringförmige Trägerkörper besitzen eine Länge von 3 bis 6 mm, einen AuBendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepaßt (vgl. EP-A 714 700).

Selbsverständlich können die Multimetalloxidaktivmassen der Festbettkatalysatoren 2 auch zu ringförmigen Trägerkatalysatoren geformt werden.

Günstige erfindungsgemäß als Festbettkatalysatoren 2 zu verwendende Multimetalloxidaktivmassen sind ferner Massen der allgemeinen Formel VI,

$$[D]_p[E]_q \qquad (VI),$$

in der die Variablen folgende Bedeutung haben:

$D =$  $MO_{12}V_{a''}Z^1_{b''}Z^2_{c''}Z^3_{d''}Z^4_{e''}Z^5_{f''}Z^6_{g''}O_{x''}$,

$E: =$  $Z^7_{12}Cu_{h''}H_{i''}O_{y''}$ ,

$Z^1 =$  W, Nb, Ta, Cr und/oder·Ce,

$Z^2 =$  Cu, Ni, Co, Fe, Mn und/oder Zn,

$Z^3 =$  Sb und/oder Bi,

$Z^4 =$  Li, Na, K, Rb, Cs und/oder H

$Z^5 =$  Mg, Ca, Sr und/oder Ba,

$Z^6 =$  Si, Al, Ti und/oder Zr,

$Z^7 =$  Mo, W, V, Nb und/oder Ta,

$a'' =$  1 bis 8,

$b'' =$  0,2 bis 5,

$c'' =$  0 bis 23,

$d'' =$  0 bis 50,

$e'' =$  0 bis 2,

$f'' =$  0 bis 5,

$g'' =$  0 bis 50,

$h'' =$  4 bis 30,

$i'' =$  0 bis 20 und

$x'',y'' =$  Zahlen, die durch die Wertigkeit und Häufigkeit der von Sauerstoff verschiedenen Element in VI bestimmt werden und

$p,q =$  von Null verschiedene Zahlen, deren Verhältnis p/q 160:1 bis 1:1 beträgt,

und die dadurch erhältlich sind, daß man eine Multimetalloxidmasse E

$$Z^7_{12}Cu_{h''}H_{i''}O_{y''} \qquad (E),$$

in feinteiliger Form getrennt vorbildet (Ausgangsmasse 1) und anschließend die vorgebildete feste Ausgangsmasse 1 in eine wäßrige Lösung, eine wäßrige Suspension oder in ein f einteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo_{12}V_{a''}Z^1_{b''}Z^2_{c''}Z^3_{d''}Z^4_{e''}Z^5_{f''}Z^6_{g''} \qquad (D),$$

enthält (Ausgangsmasse 2), im gewünschten Mengenverhältnis p:q einarbeitet, die dabei gegebenenfalls resultierende wäßrige Mischung trocknet, und die so gegebene trockene Vorläufermasse vor oder nach ihrer Formung zur gewünschten Katalysatorgeometrie bei Temperaturen von 250 bis 600°C calciniert.

Bevorzugt sind die Multimetalloxidmassen VI, bei denen die Einarbeitung der vorgebildeten festen Ausgangsmasse 1 in eine wäßrige Ausgangsmasse 2 bei einer Temperatur $\leq 70°C$ erfolgt. Eine detaillierte Beschreibung der Herstellung von Multimetalloxid VI-Aktivmassen enthalten z.B. die EP-A 668104, die DE-A 19736105 und die DE-A 19528646.

Hinsichtlich der Formgebung gilt bezüglich Multimetalloxid.VI-Aktivmassen das bei den Multimetalloxid IV-Aktivmassen Gesagte.

In anwendungstechnisch zweckmäßiger Weise erfolgt die Durchführung der zweiten Reaktionsstufe des erfindungsgemäßen Verfahrens in einem Zweizonenrohrbündelreaktor. Eine bevorzugte Variante eines für die zweite Reaktionsstufe erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903582 offenbarten Zweizonenrohrbündelreaktoren sind für eine Durchführung der zweiten Reaktionsstufe des erfindungsgemäßen Verfahrens geeignet.

D.h., in einfacher Weise befindet sich der erfindungsgemäß zu verwendende Festbettkatalysator in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Rekationszone.

D.h., in einfacher Weise umströmt ein Salzbad C diejenigen Abschnitte der Rohre (die Reaktionszone C), in welchem sich die oxidative Umsetzung des Acroleins (beim einfachen Durchgang),bis zum Erreichen eines Umsatzwertes im Bereich von 55 bis 85 mol.% vollzieht und ein Salzbad D umströmt den Abschnitt der Rohre (die Rekationszone D), in welchem sich die oxidative Anschlußumsetzung des Acroleins (beim einfachen Durchgang) bis zum Erreichen eines Umsatzwertes von wenigstens 90 mol.% vollzieht (bei Bedarf können sich an die erfindungsgemäß anzuwendenden Reaktionszonen C,D weitere Reaktionszonen anschließen, die auf individuellen Temperaturen gehalten werden).

Anwendungstechnisch zweckmäßig umfaßt die Reaktionsstufe 2 des erfindungsgemäßen Verfahrens keine weiteren Reaktionszonen: D.h., das Salzbad D umströmt zweckmäßig den Abschnitt der Rohre, in welchem sich die oxidative Anschlußumsetzung des Acroleins (beim einfachen Durchgang) bis zu einem Umsatzwert von $\geq 92$ mol.-%, oder $\geq 94$ mol.-%, oder $\geq 96$ mol.-%, oder $\geq 98$ mol.-%, und häufig sogar $\geq 99$ mol.-% oder mehr vollzieht.

Üblicherweise liegt der Beginn der Reaktionszone D hinter dem Heißpunktmaximum der Reaktionszone C. Die Temperatur des Heißpunktmaximums der Reaktionszone D liegt normalerweise unterhalb der Heißpunktmaximaltemperatur der Reaktionszone C.

Die beiden Salzbäder C, D können erfindungsgemäß relativ zur Strömungsrichtung des durch die Reaktionsrohre strömenden Reaktiongasgemisches im Gleichstrom oder im Gegenstrom durch den die Reaktionsrohre umgebenden Raum geführt werden. Selbstverständlich kann erfindungsgemäß auch in der Reaktionszone C eine Gleichströmung und in der Reaktionszone D eine Gegenströmung (oder umgekehrt) angewandt werden.

Selbstverständlich kann man in allen vorgenannten Fallkonstellationen innerhalb der jeweiligen Reaktionszone der, relativ zu den Reaktionsrohren, erfolgenden Parallelströmung der Salzschmelze noch eine Querströmung. überlagern, so daß die einzelne Reaktionszone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Üblicherweise sind in den vorgenannten Rohrbündelreaktoren (ebenso wie in den Rohrbündelreaktoren der Einzonenfahrweise) die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 22 bis 26 mm. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, daß der Abstand der zentrischen Innenachsen von zueinander nächstliegenden Kontaktrohren (die sogenannte Kontaktrohrteilung) 35 bis 45 mm beträgt (vgl. EP-B 468290).

Als Wärmeaustauschmittel eignen sich insbesondere f luide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren die Fließgeschwindigkeit innerhalb der beiden erforderlichen Wärmeaustauschmittelkreisläufe so gewählt, daß die Temperatur des Wärmeaustauschmittels von der Eintrittstelle in die Reaktionszone bis zur Austrittsstelle aus der Reaktionszone um 0 bis 15°C ansteigt. D.h., das vorgenannte $\Delta T$ kann erfindungsgemäß 1 bis 10°C, oder 2 bis 8°C oder 3 bis 6°C betragen. in die

Die Eintrittstemperatur des Wärmeaustauschmittels in die Reaktionszone C beträgt bei einer erfindungsgemäßen Zweizonenfahrweise in der zweiten Reaktionstufe normalerweise 230 bis 270°C. Die Eintrittstemperatur des Wärmeaustauschmittels in die Reaktionszone D beträgt dabei erfindungsgemäß normalerweise einerseits 250°C bis 300°C und liegt andererseits gleichzeitig wenigstens 10°C oberhalb der Eintrittstemperatur des in die Reaktionszone C eintretenden Wärmeaustauschmittels.

Bevorzugt liegt die Eintrittstemperatur des Wärmeaustauschmittels in die Reaktionszone D wenigstens 20° oberhalb der Eintrittstemperatur des in die Reaktionszone C eintretenden Wärmeaustauschmittels. Die Differenz zwischen den Eintrittstemperaturen in die Reaktionszone C bzw. D kann erfindungsgemäß somit bis zu 15°C, bis zu 25°C, bis zu 30°C, bis zu 35°C oder bis zu 40°C betragen. Normalerweise wird die vorgenannte Temperatur aber nicht mehr als 50°C betragen. Je höher die Acroleinbelastung der Katalysatorschüttung 2 beim erfindungsgemäßen Verfahren gewählt wird, um so größer sollte die Differenz zwischen der Eintrittstemperatur des Wärmeaustauschmittels in die Reaktionszone C und der Eintrittstemperatur des Wärmeaustauschmittels in die Reaktionszone D sein. Bevorzugt liegt die Eintrittstemperatur in die Reaktionszone C bei 245 bis 260°C und die Eintrittstemperatur in die Reaktionszone D bei 265 bis 285°C.

Selbstverständlich können beim erfindungsgemäßen Verfahren die beiden Reaktionszonen C, D auch in räumlich voneinander getrennten Rohrbündelreaktoren realisiert sein. Bei Bedarf kann zwischen den beiden Reaktionszonen C, D auch ein Wärmetauscher angebracht werden. Selbstredend können die beiden Reaktionszonen C, D auch als Wirbelbett gestaltet werden.

Ferner können beim erfindungsgemäßen Verfahren ganz generell auch Katalysatorschüttungen 2 verwendet werden, deren volumenspezifische Aktivität in Strömungsrichtung des Reaktionsgasgemisches kontinuierlich, abrupt oder stufenförmig zunimmt (dies kann z.B. durch Verdünnung mit Inertmaterial oder Variation der Aktivität des Multimetalloxids bewirkt werden).

Ebenso können für die erfindungsgemäße Verfahrensweise der zweiten Reaktionsstufe auch die in der EP-A 293224 und in der EP-B 257565 empfohlenen inerten Verdünnungsgase (z.B. nur Propan, oder nur Methan etc.) eingesetzt werden. Letzteres bei Bedarf auch kombiniert mit einer in Strömungsrichtung des Reaktionsgasgemisches abnehmenden volumenspezifischen Aktivität der Katalysatorschüttung 2.

Es sei an dieser Stelle auch noch einmal darauf hingewiesen, daß für eine Durchführung der zweiten Reaktionsstufe des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Reaktionszone D eine Teilmenge an die Reaktionszone C abzuführen, um gegebenenfalls ein Anwärmen eines zu kalten Reaktionsgasausgangsgemisches 2 oder eines kalten Kreisgases zu bewirken. Ferner kann die Rohrbündelcharakteristik, innerhalb einer individuellen Reaktionszone wie in der EP-A 382 098 beschrieben gestaltet werden.

Selbstredend läßt sich das erfindungsgemäße Verfahren auch in einem einzigen Zweizonenrohrbündelreaktor, wie er z.B. in der DE-C 2830765, der EP-A 911313 sowie in der EP-A 383224 beschrieben ist, so realisieren, daß die erste Reaktionsstufe in der ersten Reaktionszone und die zweite Reaktionsstufe in der zweiten Reaktionszone des Zweizonenrohrbündelreaktors verwirklicht wird.

Das erfindungsgemäße Verfahren eignet sich insbesondere für eine kontinuierliche Durchführung. Es überrascht, daß es bei einmaligem Durchgang bei hoher Eduktbelastung der Festbettkatalysatoren gute Selektivitäten der Wertproduktbildung ermöglicht.

Bei dem erfindungsgemäßen Verfahren wird keine reine Acrylsäure sondern ein Gemisch erhalten, von dessen Nebenkomponenten die Acrylsäure in an sich bekannter Weise (z.B. rektifikativ und/oder kristallisativ) abgetrennt werden kann. Nicht umgesetztes Acrolein, Propen sowie verwendetes und/oder im Verlauf der Reaktion gebildetes inertes Verdünnungsgas können in die Gasphasenoxidation rückgeführt werden. Bei der erfindungsgemäßen zweistufigen, von Propen ausgehenden Gasphasenoxidation erfolgt die Rückführung zweckmäßigerweise in die erste Oxidationsstufe. Natürlich kann die erfindungsgemäße verfahrensweise bei Bedarf auch im Fall konventioneller Propenlasten angewendet werden.

Im übrigen sind in dieser Schrift Umsatz, Selektivität und Verweilzeit, falls nichts anderes erwähnt wird, wie folgt definiert:

$$\text{Umsatz an Edukt (\%)} = \frac{\text{Molzahl umgesetztes Edukt}}{\text{Molzahl eingesetztes Edukt}} \text{X } 100$$

$$\text{Selektivität der Produktbildung} = \frac{\text{Molzahl Edukt umgesetzt zu Produkt}}{\text{Molzahl umgesetztes Edukt}} \text{X } 100$$

$$\text{Verweilzeit (sec.)} = \frac{\text{mit Katalysator gefülltes Leervolumen des Reaktors (1)}}{\text{durchgesetzte Menge Reaktions-gasausgangsgemisch (Nl/h)}} \text{X } 3600$$

Beispiele und Vergleichsbeispiele

a) Herstellung eines erfindungsgemäßen Festbettkatalysators 1

1. Herstellung einer Ausgangsmasse 1

In 775 kg einer wäßrigen salpetersauren Wismutnitratlösung (11,2 Gew.-% Bi, freie Salpetersäure 3 bis 5 Gew.-%; Massendichte: 1,22 bis 1,27 g/ml) wurden bei 25°C portionsweise 209,3 kg Wolframsäure (72,94 Gew.-% W) eingerührt. Das resultierende wäßrige Gemisch wurde anschließend noch 2 h bei 25°C gerührt und anschließend sprühgetrocknet.

Die Sprühtrocknung erfolgte in einem Drehscheibensprühturm im Gleichstrom bei einer Gaseintrittstemperatur von $300 \pm 10°C$ und einer Gasaustrittstemperatur von $100 \pm 20°C$. Das erhaltene Sprühpulver wurde anschließend bei einer Temperatur im Bereich von 780 bis 810°C calciniert (im luftdurchströmten Drehrohrofen (1,54 $m^3$ Innenvolumen, 200 $Nm^3$ Luft/h)). wesentlich bei der genauen Einstellung der Calcinationstemperatur ist, daß sie an der angestrebten Phasenzusammensetzung des Calcinationsprodukts orientiert zu erfolgen hat. Gewünscht sind die Phasen $WO_3$ (monoklin) und $Bi_2W_2O_9$, unerwünscht ist das Vorhandensein von $\gamma$-$Bi_2WO_6$ (Russellit). Sollte daher nach der Calcination die Verbindung $\gamma$-$Bi_2WO_6$ anhand eines Reflexes im Pulverröntgendiffraktogramm bei einem Reflexwinkel von $2\ominus = 28,4°$ (CuK$\alpha$-Strahlung) noch nachweisbar sein, so ist die Präparation zu wiederholen und die Calcinationstemperatur innerhalb des angegebenen Temperaturbereichs zu erhöhen, bis das Verschwinden des Reflexes erreicht wird. Das so erhaltene vorgebildete calcinierte Mischoxid wurde gemahlen, so daß der $X_{50}$-Wert (vgl. Ullmann's Encyclopedia of Industrial Chemistry, $6^{th}$ Edition (1998) Electronic Release, Kapitel 3.1.4 oder DIN 66141) der resultierenden Körnung 5 µm betrug. Das Mahlgut wurde dann mit 1 Gew.-% (bezogen auf das Mahlgut) feinteiligem $SiO_2$ (Rüttelgewicht 150 g/l; $X_{50}$-Wert der $SiO_2$-Partikel betrug 10 µm, die BET-Oberfläche betrug 100 $m^2$/g) vermischt.

2. Herstellung einer Ausgangsmasse 2

Eine Lösung A wurde hergestellt indem man bei 60°C unter Rühren in 600 l Wasser 213 kg Ammoniumheptamolybdat löste und die resultierende Lösung unter Aufrechterhaltung der 60°C und Rühren mit 0,97 kg einer 20°C aufweisenden wäßrigen Kaliumhydroxidlösung (46,8 Gew.-% KOH) versetzte.

Eine Lösung B wurde hergestellt indem man bei 60°C in 262,9 kg einer wäßrigen Cobaltnitratlösung (12,4 Gew.-% Co) 116,25 kg einer wäßrigen Eisennitratlösung (14,2 Gew.-% Fe) eintrug. Anschließend wurde unter Aufrechterhaltung der 60°C die Lösung B über einen Zeitraum von 30 Minuten kontinuierlich in die vorgelegte Lösung A gepumpt. Anschließend wurde 15 Minuten bei 60°C gerührt. Dann wurden dem resultierenden wäßrigen Gemisch 19,16 kg eines Kieselgels (46,80 Gew.-% $SiO_2$, Dichte: 1,36 bis 1,42 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Gleichstrom sprühgetrocknet (Gaseintrittstemperatur: $400 \pm 10°C$, Gasaustrittstemperatur: $140 \pm 5°C$). Das resultierende Sprühpulver wies einen Glühverlust von ca. 30 Gew.-% auf (3 h bei 600°C glühen).

3. Herstellung der Multimetalloxidaktivmasse

Die Ausgangsmasse 1 wurde mit der Ausgangsmasse 2 in der für eine Multimetalloxidaktivmasse der Stöchiometrie

$$[Bi_2W_2O_9 \cdot 2WO_3]_{0,5}[Mo_{12}Co_{5,5}Fe_{2,94}Si_{1,59}K_{0,08}O_x]_1$$

erforderlichen Menge homogen vermischt. Bezogen auf die vorgenannte Gesamtmasse wurden zusätzlich 1,5 Gew.-% feinteiliges Graphit (Siebanalyse: min. 50 Gew.-% < 24 µm, max. 10 Gew.-% > 24 µm und < 48 µm, max. 5 Gew.-

% > 48 µm, BET-Oberfläche: 6 bis 13 m$^2$/g) homogen eingemischt. Das resultierende Trockengemisch wurde zu Hohlzylindern mit 3 mm Länge, 5 mm Außendurchmesser und 1,5 mm Wandstärke verpreßt und anschließend wie folgt thermisch behandelt.

Im Luft durchströmten Muffelofen (60 l Innenvolumen, 1 l/h Luft pro Gramm Aktivmassevorläufermasse) wurde mit einer Aufheizrate von 180°C/h zunächst von Raumtemperatur (25°C) auf 190°C aufgeheizt. Diese Temperatur wurde für 1 h aufrechterhalten und dann mit einer Aufheizrate von 60°C/h auf 210°C erhöht. Die 210°C wurden wiederum während 1 h aufrechterhalten, bevor sie mit einer Aufheizrate von 60°C/h, auf 230°C erhöht wurden. Diese Temperatur wurde ebenfalls 1 h aufrechterhalten bevor sie, wiederum mit einer Aufheizrate von 60°C/h, auf 265°C erhöht wurde. Die 265°C wurden anschließend ebenfalls während 1 h aufrechterhalten. Danach wurde zunächst auf Raumtemperatur abgekühlt und damit die Zersetzungsphase im wesentlichen abgeschlossen. Dann wurde mit einer Aufheizrate von 180°C/h auf 465°C erhitzt und diese Calcinationstemperatur während 4 h aufrechterhalten. Eine Schüttung aus den resultierenden Vollkatalysatorringen bildete einen erfindungsgemäßen Festbettkatalysator 1.

b) Herstellung eines erfindungsgemäßen Festbettkatalysators 2

1. Herstellung der katalytisch aktiven Oxidmasse Mo$_{12}$V$_3$W$_{1,2}$Cu$_{2,4}$O$_n$

190 g Kupfer(II)acetatmonohydrat wurden in 2700 g Wasser zu einer Lösung I gelöst. In 5500 g Wasser wurden bei 95°C nacheinander 860 g Ammoniumheptamolybdattetrahydrat, 143 g Ammoniummetavanadat und 126 g Ammoniumparawolframatheptahydrat zu einer Lösung II gelöst. Anschließend wurde die Lösung I auf einmal in die Lösung II eingerührt und anschließend soviel einer 25gew.-%igen wäßrigen NH$_3$-Lösung zugesetzt, bis wieder eine Lösung entstand. Diese wurde bei einer Austrittstemperatur von 110°C sprühgetrocknet. Das resultierende Sprühpulver wurde je kg Pulver mit 0,25 kg einer 30gew.-%igen wäßrigen Essigsäurelösung mit einem Kneter der Fa. Werner & Pfleiderer vom Typ ZS1-80 verknetet und anschließend bei einer Temperatur von 110°C während 10 h im Trockenschrank getrocknet.

700 g des so erhaltenen Katalysatorvorläufers wurden in einem Luft/Stickstoffgemisch [(200 l N$_2$/15 l Luft)/h] in einem Drehrohrofen (50 cm lang, 12 cm Innendurchmesser) calciniert. Im Rahmen der Calcinierung wurde die Knetmasse zunächst innerhalb von einer Stunde von Raumtemperatur (ca. 25°C) kontinuierlich auf 325°C erhitzt. Anschließend wurde während 4 h auf dieser Temperatur gehalten. Dann wurde innerhalb von 15 min auf 400°C erwärmt, bei dieser Temperatur während 1 h gehalten und dann auf Raumtemperatur abgekühlt.

Das calcinierte katalytisch aktive Material wurde zu einem feinteiligen Pulver gemahlen, von dem 50 % der Pulverpartikel ein Sieb der Maschenweite 1 bis 10 µm passierten und dessen Anteil an Partikel mit einer Längstausdehnung oberhalb von 50 µm weniger als 1 % betrug.

2. Schalenkatalysatorherstellung

28 kg ringförmiger Trägerkörper (7 mm Außendurchmesser, 3 mm Länge, 4 mm Innendurchmesser, Steatit, mit einer Oberflächenrauhigkeit Rz gemäß EP-B 714700 von 45 µm und mit einem auf das Volumen der Trägerkörper bezogenen Porengesamtvolumen ≤ 1 Vol.-%, Hersteller: Caramtec DE) wurden in einem Dragierkessel (Neigungswinkel 90°; Hicoater der Fa. Lödige, DE) von 200 l Innenvolumen gefüllt. Anschließend wurde der Dragierkessel mit 16 U/min in Rotation versetzt. Über eine Düse wurden innerhalb von 25 min 2000 g einer aus 75 Gew.-% H$_2$O und 25 Gew.-% Glycerin bestehenden wäßrigen Lösung auf die Trägerkörper aufgesprüht. Gleichzeitig wurden im selben zeitraum 7 kg des katalytisch aktiven Oxidpulvers aus a) über eine Schüttelrinne außerhalb des Sprühkegels der Zerstäuberdüse kontinuierlich zudosiert. Während der Beschichtung wurde das zugeführte Pulver vollständig auf die Oberfläche der Trägerkörper aufgenommen, eine Agglomeration der feinteiligen oxidischen Aktivmasse wurde nicht beobachtet. Nach beendeter Zugabe von Pulver und wäßriger Lösung wurde bei einer Drehgeschwindigkeit von 2 Umdrehungen/min 20 min. 110°C heiße Luft in den Dragierkessel geblasen. Anschließend wurde noch 2 h bei 250°C in ruhender Schüttung (Hordenofen) unter Luft getrocknet. Es wurden ringförmige Schalenkatalysatoren erhalten, deren Anteil an oxidischer Aktivmasse, bezogen auf die Gesamtmasse, 20 Gew.-% betrug. Die Schalendicke lag, sowohl über die Oberfläche eines Trägerkörpers als auch über die Oberfläche verschiedener Trägerkörper betrachtet, bei 230 ± 25 µm. Eine Schüttung aus den resultierenden Schalenkatalysatorringen bildete einen erfindungsgemäßen Festbettkatalysator 2.

c) Herstellung eines kugelförmigen Vergleichsfestbettschalenkatalysators 1

1. Herstellung einer Ausgangsmasse 1

In 775 kg einer wäßrigen salpetersauren Wismutnitratlösung (11,2 Gew.-% Bi, freie Salpetersäure 3 bis 5 Gew.-%; Massendichte: 1,22 bis 1,27 g/ml) wurden bei 25°C portionsweise 209,3 kg Wolframsäure (72,94 Gew.-% W) eingerührt. Das resultierende wäßrige Gemisch wurde anschließend noch 2 h bei 25°C gerührt und anschließend sprühgetrocknet.

Die Sprühtrocknung erfolgte in einem Drehscheibensprühturm im Gleichstrom bei einer Gaseintrittstemperatur von $300 \pm 10°$C und einer Gasaustrittstemperatur von $100 \pm 10°$C. Das erhaltene Sprühpulver wurde anschließend bei einer Temperatur im Bereich von 780 bis 810°C calciniert (im luftdurchströmten Drehrohrofen (1,54 m$^3$ Innenvolumen, 200 Nm$^3$ Luft/h)). Wesentlich bei der genauen Einstellung der Calcinationstemperatur ist, daß sie an der angestrebten Phasenzusammensetzung des Calcinationsprodukts orientiert zu erfolgen hat. Gewünscht sind die Phasen $WO_3$ (monoklin) und $Bi_2W_2O_9$, unerwünscht ist das Vorhandensein von $\gamma$-$Bi_2WO_6$ (Russellit). Sollte daher nach der Calcination die Verbindung $\gamma$-$Bi_2WO_6$ anhand eines Reflexes im Pulverröntgendiffraktogramm bei einem Reflexwinkel von $2\ominus$ =.28,4° (CuKa-Strahlung) noch nachweisbar sein, so ist die Präparation zu wiederholen und die Calcinationstemperatur innerhalb des angegebenen Temperaturbereichs zu erhöhen, bis das Verschwinden des Reflexes erreicht wird. Das so erhaltene vorgebildete calcinierte Mischoxid wurde gemahlen, so daß der $X_{50}$-Wert (vgl. Ullmann's Encyclopedia of Industrial Chemistry, 6$^{th}$ Edition (1998) Electronic Release, Kapitel 3.1.4 oder DIN 66141) der resultierenden Körnung 5 µm betrug. Das Mahlgut wurde dann mit 1 Gew.-% (bezogen auf das Mahlgut) feinteiligem $SiO_2$ (Rüttelgewicht 150 g/l; $X_{50}$-Wert der $SiO_2$-Partikel betrug 10 µm, die BET-Oberfläche betrug 100 m$^2$/g) vermischt.

2. Herstellung einer Ausgangsmasse 2

Eine Lösung A wurde hergestellt indem man bei 60°C unter Rühren in 600 l Wasser 213 kg Ammoniumheptamolybdat löste und die resultierende Lösung unter Aufrechterhaltung der 60°C und Rühren mit 0,97 kg einer 20°C aufweisenden wäßrigen Kaliumhydroxidlösung (46,8 Gew.-% KOH) versetzte.

Eine Lösung B wurde hergestellt indem man bei 60°C in 262,9 kg einer wäßrigen Cobaltnitratlösung (12,4 Gew.-% Co) 116,25 kg einer wäßrigen Eisennitratlösung (14,2 Gew.-% Fe) eintrug. Anschließend wurde unter Aufrechterhaltung der 60°C die Lösung B über einen Zeitraum von 30 Minuten kontinuierlich in die vorgelegte Lösung A gepumpt. Anschließend wurde 15 Minuten bei 60°C gerührt. Dann wurden dem resultierenden wäßrigen Gemisch 19,16 kg eines Kieselgels (46,80 Gew.-% $SiO_2$, Dichte: 1,36 bis 1,42 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Gegenstrom sprühgetrocknet (Gaseintrittstemperatur: $400\pm 10°$C, Gasaustrittstemperatur: $140\pm 5°$C). Das resultierende Sprühpulver wies einen Glühverlust von ca. 30 Gew.-%.auf (3 h bei 600°C glühen).

3. Herstellung der Multimetalloxidaktivmasse

Die Ausgangsmasse 1 wurde mit der Ausgangsmasse 2 in der für eine Multimetalloxidaktivmasse der Stöchiometrie

$$[Bi_2W_2O_9 \cdot 2WO_3]_{0,5}[Mo_{12}Co_{5,5}Fe_{2,94}Si_{1,59}K_{0,0,8}O_x]_1$$

erforderlichen Menge homogen vermischt. Bezogen auf die vorgenannte Gesamtmasse wurden zusätzlich 1,5 Gew.-% feinteiliges Graphit (Siebanalyse: min. 50 Gew.-% < 24 µm, max. 10 Gew.-% > 24 µm und < 48 µm, max. 5 Gew.-% > 48 µm, BET-Oberfläche: 6 bis 13 m$^2$/g) homogen eingemischt. Das resultierende Trockengemisch wurde zu Hohlzylindern mit 3 mm Länge, 5 mm Außendurchmesser und 1,5 mm Wandstärke verpreßt und anschließend wie folgt thermisch behandelt.

Im Luft durchströmten Muffelofen (60 1 Innenvolumen, 1 l/h Luft pro Gramm Aktivmassevorläufermasse) wurde mit einer Aufheizrate von 180°C/h zunächst von Raumtemperatur (25°C) auf 190°C aufgeheizt. Diese Temperatur wurde für 1 h aufrechterhalten und dann mit einer Aufheizrate von 60°C/h auf 210°C erhöht. Die 210°C wurden wiederum während 1 h aufrechterhalten, bevor sie mit einer Aufheizrate von 60°C/h, auf 230°C erhöht wurden. Diese Temperatur wurde ebenf alls 1 h aufrechterhalten bevor sie, wiederum mit einer Aufheizrate von 60°C/h, auf 265°C erhöht wurde. Die 265°C wurden anschließend ebenfalls während 1 h aufrechterhalten. Danach wurde zunächst auf Raumtemperatur abgekühlt und damit die Zersetzungsphase im wesentlichen abgeschlossen. Dann wurde mit einer Aufheizrate von 180°C/h auf 465°C erhitzt und diese Calcinationstemperatur während 4 h aufrechterhalten.

Das calcinierte katalytisch aktive Material wurde zu einem feinteiligen Pulver gemahlen, von dem 50 % der Pulverpartikel ein Sieb der Maschenweite 1 bis 10 µm passierten und dessen Anteil an Partikel mit einer Längstausdehnung oberhalb von 50 µm weniger als 1 % betrug.

4. Schalenkatalysatorherstellung

30 kg kugelförmiger Trägerkörper (4 - 5 mm Durchmesser mit einer Oberflächenrauhigkeit Rz gemäß EP-B 714700 von 45 μm und mit einem auf das Volumen der Trägerkörper bezogenen Porengesamtvolumen ≤ 1 Vol.-%, Hersteller: Ceramtec DE) wurden in einem Dragierkessel (Neigungswinkel 90°; Hicoater der Fa. Lödige, DE) von 200 l Innenvolumen gefüllt. Anschließend wurde der Dragierkessel mit 16 U/min in Rotation versetzt. Über eine Düse wurden innerhalb von 25 min 2500 g einer aus 75 Gew.-% $H_2O$ und 25 Gew.-% Glycerin bestehenden wäßrigen Lösung auf die Trägerkörper aufgesprüht. Gleichzeitig wurden im selben Zeitraum 13 kg des katalytisch aktiven Oxidpulvers aus a) über eine Schüttelrinne außerhalb des Sprühkegels der Zerstäuberdüse kontinuierlich zudosiert. Während der Beschichtung wurde das zugeführte Pulver vollständig auf die Oberfläche der Trägerkörper aufgenommen, eine Agglomeration der feinteiligen oxidischen Aktivmasse wurde nicht beobachtet. Nach beendeter Zugabe von Pulver und wäßriger Lösung wurde bei einer Drehgeschwindigkeit von 2 Umdrehungen/min 20 min. 110°C heiße Luft in den Dragierkessel geblasen. Anschließend wurde noch 2 h bei 250°C in ruhender Schüttung (Hordenofen) unter Luft getrocknet. Es wurden kugelförmige Schalenkatalysatoren erhalten, deren Anteil an oxidischer Aktivmasse, bezogen auf die Gesamtmasse, 30 Gew.-% betrug. Die Schalendicke lag, sowohl über die Oberfläche eines Trägerkörpers als auch über die Oberfläche verschiedener Trägerkörper betrachtet, bei 280 ± 25 μm. Eine Schüttung aus den resultierenden Schalenkatalysatorkugeln bildete den kugelförmigen Vergleichsfestbettschalenkatalysator 1.

d) Herstellung eines kugelförmigen Vergleichsfestbettschalenkatalysators 2

1. Herstellung der katalytisch aktiven Oxidmasse $Mo_{12}V_3W_{1,2}Cu_{2,4}O_n$

190 g Kupfer(II)acetatmonohydrat wurden in 2700 g Wasser zu einer Lösung I gelöst. In 5500 g Wasser wurden bei 95°C nacheinander 860.g Ammoniumheptamolybdattetrahydrat, 143 g Ammoniummetavanadat und 126 g Ammoniumparawolframatheptahydrat zu einer Lösung II gelöst. Anschließend wurde die Lösung I auf einmal in die Lösung II eingerührt und anschließend soviel einer 25gew.-%igen wäßrigen $NH_3$-Lösung zugesetzt, bis wieder eine Lösung entstand. Diese wurde bei einer Austrittstemperatur von 110°C sprühgetrocknet. Das resultierende Sprühpulver wurde je kg Pulver mit 0,25 kg einer 30gew.-%igen wäßrigen Essigsäurelösung mit einem Kneter der Fa. Werner & Pfleiderer vom Typ ZS1-80 verknetet und anschließend bei einer Temperatur von 110°C während 10 h im Trockenschrank getrocknet.

700 g des so erhaltenen Katalysatorvorläufers wurden in einem Luft/Stickstoffgemisch [(200 l $N_2$/15 l Luft)/h] in einem Drehrohrofen (50 cm lang, 12 cm Innendurchmesser) calciniert. Im Rahmen der Calcinierung wurde die Knetmasse zunächst innerhalb von einer Stunde von Raumtemperatur (ca. 25°C) kontinuierlich auf 325°C erhitzt. Anschließend wurde während 4 h auf dieser Temperatur gehalten. Dann wurde innerhalb von 15 min auf 400°C erwärmt, bei dieser Temperatur während 1 h gehalten und dann auf Raumtemperatur abgekühlt.

Das calcinierte katalytisch aktive Material wurde zu einem feinteiligen Pulver gemahlen, von dem 50 % der Pulverpartikel ein Sieb der Maschenweite 1 bis 10 μm passierten und dessen Anteil an Partikel mit einer Längstausdehnung oberhalb von 50 μm weniger als 1 % betrug.

2. Schalenkatalysatorherstellung

30 kg kugelförmiger Trägerkörper (4-5 mm Durchmesser, Steatit, mit einer Oberflächenrauhigkeit Rz gemäß EP-B 714700 von 45 μm und mit einem auf das Volumen der Trägerkörper bezogenen Porengesamtvolumen ≤ 1 Vol.-%, Hersteller: Caramtec DE) wurden in einem Dragierkessel (Neigungswinkel 90°; Hicoater der Fa. Lödige, DE) von 200 l Innenvolumen gefüllt. Anschließend wurde der Dragierkessel mit 16 U/min in Rotation versetzt. Über eine Düse wurden innerhalb von 25 min 1600 g einer wäßrigen Lösung auf die Trägerkörper aufgesprüht. Gleichzeitig wurden im selben Zeitraum 5,3 kg des katalytisch aktiven Oxidpulvers aus a) über eine Schüttelrinne außerhalb des Sprühkegels der Zerstäuberdüse kontinuierlich zudosiert. Während der Beschichtung wurde das zugeführte Pulver vollständig auf die Oberfläche der Trägerkörper aufgenommen, eine Agglomeration der feinteiligen oxidischen Aktivmasse wurde nicht beobachtet. Nach beendeter Zugabe von Pulver und wäßriger Lösung wurde bei einer Drehgeschwindigkeit von 2 Umdrehungen/min 20 min. 110°C heiße Luft in den Dragierkessel geblasen. Es wurden kugelförmige Schalenkatalysatoren erhalten, deren Anteil an oxidischer Aktivmasse, bezogen auf die Gesamtmasse, 15 Gew.-% betrug. Die Schalendicke lag, sowohl über die Oberfläche eines Trägerkörpers als auch über die Oberfläche verschiedener Trägerkörper betrachtet, bei 210 ± 5 μm. Eine Schüttung aus den resultierenden Schalenkatalysatorkugeln bildete den kugelförmigen vergleichsfestbettschalenkatalysator 2.

e) Herstellung eines Vergleichsfestbettvollzylinderkatalysators 1

1. Herstellung einer Ausgangsmasse 1

In 775 kg einer wäßrigen salpetersauren Wismutnitratlösung (11,2 Gew.-% Bi, freie Salpetersäure 3 bis 5 Gew.-%; Massendichte: 1,22 bis 1,27 g/ml) wurden bei 25°C portionsweise 209,3 kg wolframsäure (72,94 Gew.-% W) eingerührt. Das resultierende wäßrige Gemisch wurde anschließend noch 2 h bei 25°C gerührt und anschließend sprühgetrocknet.

Die Sprühtrocknung erfolgte in einem Drehscheibensprühturm im Gleichstrom bei einer Gaseintrittstemperatur von $300 \pm 10°C$ und einer Gasaustrittstemperatur von $100 \pm 10°C$. Das erhaltene Sprühpulver wurde anschließend bei einer Temperatur im Bereich von 780 bis 810°C calciniert (im luftdurchströmten Drehrohrofen (1,54 m$^3$ Innenvolumen, 200 Nm$^3$ Luft/h)). Wesentlich bei der genauen Einstellung der Calcinationstemperatur ist, daß sie an der angestrebten Phasenzusammensetzung des Calcinationsprodukts orientiert zu erfolgen hat. Gewünscht sind die Phasen $WO_3$ (monoklin) und $Bi_2W_2O_9$, unerwünscht ist das Vorhandensein von $\gamma$-$Bi_2WO_6$ (Russellit). Sollte daher nach der Calcination die Verbindung $\gamma$-$Bi_2WO_6$ anhand eines Reflexes im Pulverröntgendiffraktogramm bei einem Reflexwinkel von $2\ominus = 28,4°$ (CuK$\alpha$-Strahlung) noch nachweisbar sein, so ist die Präparation zu wiederholen und die Calcinationstemperatur innerhalb des angegebenen Temperaturbereichs zu erhöhen, bis das Verschwinden des Reflexes erreicht wird. Das so erhaltene vorgebildete calcinierte Mischoxid wurde gemahlen, so daß der $X_{50}$-Wert (vgl. Ullmann's Encyclopedia of Industrial Chemistry, 6$^{th}$ Edition (1998) Electronic Release, Kapitel 3.1.4 oder DIN 66141) der resultierenden Körnung 5 µm betrug. Das Mahlgut wurde dann mit 1 Gew.-% (bezogen auf das Mahlgut) feinteiligem $SiO_2$ (Rüttelgewicht 150 g/l; $X_{50}$-Wert der $SiO_2$-Partikel betrug 10 µm, die BET-Oberfläche betrug 100 m$^2$/g) vermischt.

2. Herstellung einer Ausgangsmasse 2

Eine Lösung A wurde hergestellt indem man bei 60°C unter Rühren in 600 l Wasser 213 kg Ammoniumheptamolybdat löste und die resultierende Lösung unter Aufrechterhaltung der 60°C und Rühren mit 0,97 kg einer 20°C aufweisenden wäßrigen Kaliumhydroxidlösung (46,8 Gew.-% KOH) versetzte.

Eine Lösung B wurde hergestellt indem man bei 60°C in 262,9 kg einer wäßrigen Cobaltnitratlösung (12,4 Gew.-% Co) 116,25 kg einer wäßrigen Eisennitratlösung (14,2 Gew.-% Fe) eintrug. Anschließend wurde unter Aufrechterhaltung der 60°C die Lösung B über einen Zeitraum von 30 Minuten kontinuierlich in die vorgelegte Lösung A gepumpt. Anschließend wurde 15 Minuten bei 60°C kg gerührt. Dann wurden dem resultierenden wäßrigen Gemisch 19,16 kg eines Kieselgels (46,80 Gew.-% $SiO_2$, Dichte: 1,36 bis 1,42 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Gleichstrom sprühgetrocknet (Gaseintrittstemperatur: $400 \pm 10°C$, Gasaustrittstemperatur: $140 \pm 5°C$). Das resultierende Sprühpulver wies einen Glühverlust von ca. 30 Gew.-% auf (3 h bei 600°C glühen).

3. Herstellung der Multimetalloxidaktivmasse

Die Ausgangsmasse 1 wurde mit der Ausgangsmasse 2 in der für eine Multimetalloxidaktivmasse der Stöchiometrie

$$[Bi_2W_2O_9 \cdot 2WO_3]_{0,5}[Mo_{12}Co_{5,5}Fe_{2,94}Si_{1,59}K_{0,08}O_x]_1$$

erforderlichen Menge homogen vermischt. Bezogen auf die vorgenannte Gesamtmasse wurden zusätzlich 1,5 Gew.-% feinteiliges Graphit (Siebanalyse: min. 50 Gew.-% < 24 µm, max. 10 Gew.-% > 24 µm und < 48 µm, max. 5 Gew.-% > 48 µm, BET-Oberfläche: 6 bis 13 m$^2$/g) homogen eingemischt. Das resultierende Trockengemisch wurde zu Vollzylindern mit 3 mm Länge und 5 mm Außendurchmesser verpreßt und anschließend wie folgt thermisch behandelt.

Im Luft durchströmten Muffelofen (60 l Innenvolumen, 1 l/h Luft pro Gramm Aktivmassevorläufermasse) wurde mit einer Aufheizrate von 150°C/h zunächst von Raumtemperatur (25°C) auf 180°C aufgeheizt. Diese Temperatur wurde für 1,5 h aufrechterhalten und dann mit einer Aufheizrate von 60°C/h auf 200°C erhöht. Die 200°C wurden wiederum während 1,5 h aufrechterhalten, bevor sie mit einer Aufheizrate von 60°C/h auf 220°C erhöht wurden. Diese Temperatur wurde ebenfalls 1,5 h aufrechterhalten bevor sie, wiederum mit einer Aufheizrate von 60°C/h, auf 250°C erhöht wurde. Die 250°C wurden anschließend ebenfalls während 1,5 h aufrechterhalten. Danach wurde zunächst auf Raumtemperatur abgekühlt und damit die Zersetzungsphase im wesentlichen abgeschlossen. Dann wurde mit einer Aufheizrate von 180°C/h auf 465°C erhitzt und diese Calcinationstemperatur während 4 h aufrechterhalten. Eine Schüttung aus dem resultierenden Vollkatalysator bildete den Vergleichsfestbettvollzylinderkatalysator 1.

f) Herstellung eines Vergleichsfestbettvollzylinderkatalysators 2

1. Herstellung der katalytisch aktiven Oxidmasse $Mo_{12}V_3W_{1,2}Cu_{2,4}O_n$

190 g Kupfer(II)acetatmonohydrat wurden in 2700 g Wasser zu einer Lösung I gelöst. In 5500 g Wasser wurden bei 95°C nacheinander 860 g Ammoniumheptamolybdattetrahydrat, 143 g Ammoniummetavanadat und 126 g Ammoniumparawolframatheptahydrat zu einer Lösung II gelöst. Anschließend wurde die Lösung I auf einmal in die Lösung II eingerührt und anschließend soviel einer 25gew.-%igen wäßrigen $NH_3$-Lösung zugesetzt, bis wieder eine Lösung entstand. Diese wurde bei einer Austrittstemperatur von 110°C sprühgetrocknet. Das resultierende Sprühpulver wurde je kg Pulver mit 0,25 kg einer 30gew.-%igen wäßrigen Essigsäurelösung mit einem Kneter der Fa. Werner & Pfleiderer vom Typ ZS1-80 verknetet und anschließend bei einer Temperatur von 110°C während 10 h im Trockenschrank getrocknet.

700 g des so erhaltenen Katalysatorvorläufers wurden in einem Luft/Stickstoffgemisch [(200 l $N_2$/15 l Luft)/h] in einem Drehrohrofen (50 cm lang, 12 cm Innendurchmesser) calciniert. Im Rahmen der Calcinierung wurde die Knetmasse zunächst innerhalb von einer Stunde von Raumtemperatur (ca. 25°C) kontinuierlich auf 325°C erhitzt. Anschließend wurde während 4 h auf dieser Temperatur gehalten. Dann wurde innerhalb von 15 min auf 400°C erwärmt, bei dieser Temperatur während 1 h gehalten und dann auf Raumtemperatur abgekühlt.

Das calcinierte katalytisch aktive Material wurde zu einem feinteiligen Pulver gemahlen, von dem 50 % der Pulverpartikel ein Sieb der Maschenweite 1 bis 10 µm passierten und dessen Anteil an Partikel mit einer Längstausdehnung oberhalb von 50 µm weniger als 1 % betrug.

Das so erhaltene katalytisch aktive Material wurde nach Zumischung von 3 Gew.-% (bezogen auf die Aktivmasse) Graphit zu Voll zylindern mit 3 mm Länge und 5 mm Außendurchmesser verpreßt.

Eine Schüttung aus den resultierenden Vollkatalysatoren bildete den Vergleichsfestbettvollzylinderkatalysator 2.

g) Gasphasenkatalytische Oxidation von Propen zu Acrylsäure

1. Die erste Reaktionsstufe

Ein erstes Reaktionsrohr (V2A Stahl; 30 mm AuBendurchmesser; 2 mm Wandstärke; 26 mm Innendurchmesser, Länge: 439 cm, sowie ein in der Reaktionsrohrmitte zentriertes Thermorohr (4 mm Außendurchmesser) zur Aufnahme eines Thermoelements mit dem die Temperatur im Reaktionsrohr ermittelt werden kann) wurde von unten nach oben auf einem Kontaktstuhl (44 cm Länge) zunächst auf einer Länge von 30 cm mit eine rauhe Oberfläche aufweisenden Steatitkugeln (4 bis 5 mm Durchmesser; Inertmaterial zum Erwärmen des Reaktionsgasausgangsgemisches 1) und anschließend auf einer Länge von 300 cm mit dem in a) (bzw. in c) oder in e)) hergestellten Festbettkatalysator 1 beschickt, bevor die Beschickung auf einer Länge von 30 cm mit den vorgenannten Steatitkugeln als Nachschüttung abgeschlossen wurde. Die verbleibenden 35 cm Kontaktrohr wurden leer belassen.

Der Teil des ersten Reaktionsrohres, der mit Feststoff beschickt war, wurde mittels 12 zylinderförmig um das Rohr aufgegossenen Aluminium-Blöcken von je 30 cm Länge, die durch elektrische Heizbänder beheizt wurden, thermostatisiert (Vergleichsversuche mit einem entsprechenden mittels eines stickstoffgeperlten Salzbades beheizten Reaktionsrohr zeigten, daß die Aluminiumblock-Thermostatisierung eine Salzbad-Thermostatisierung zu simulieren vermag). Die ersten sechs Aluminiumblöcke in Strömungsrichtung definierten eine Reaktionszone A und die verbleibenden Aluminiumblöcke definierten eine Reaktionszone B. Die an Feststoff freien Enden des Reaktionsrohres wurden mit unter erhöhtem Druck befindlichem Wasserdampf auf 220°C gehalten.

2. Die zweite Reaktionsstufe

Ein zweites Reaktionsrohr (V2A Stahl; 30 mm Außendurchmesser; 2 mm Wandstärke; 26 mm Innendurchmesser, Länge: 439 cm sowie ein in der Reaktionsrohrmitte zentriertes Thermo-Rohr (4 mm Außendurchmesser) zur Aufnahme eines Thermoelements mit dem die Temperatur im Reaktionsrohr ermittelt werden kann) wurde von unten nach oben auf einem Kontaktstuhl (44 cm Länge) zunächst auf einer Länge von 30 cm mit eine rauhe Oberfläche aufweisenden Steatitkugeln (4 bis 5 mm Durchmesser; Inertmaterial zum Temperieren des Reaktionsgasausgangsgemisches 2) und anschließend auf einer Länge von 300 cm mit dem in b) (bzw. in d) oder f)) hergestellten Festbettkatalysator 2 beschickt, bevor die Beschickung auf einer Länge von 30 cm mit den vorgenannten Steatitkugeln als Vorschüttung abgeschlossen wurde. Die verbleibenden 35 cm Kontaktrohr wurden leer belassen.

Der Teil des zweiten Reaktionsrohres, der mit Feststoff beschickt war, wurde mittels 12 zylinderförmig um das Rohr aufgegossenen Aluminium-Blöcken von je 30 cm Länge thermostatisiert (Vergleichsversuche mit einem entsprechenden mittels eines stickstoffgeperlten Salzbades beheizten Reaktionsrohr zeigten, daß die Aluminiumblock-Thermostatisierung eine Salzbad-Thermostatisierung zu simulieren vermag). Die ersten sechs Aluminiumblöcke in Strömungsrichtung definierten eine Reaktionszone C und die verbleibenden sechs Aluminiumblöcke definierten eine Reaktionszone D. Die an Feststoff freien Enden des Reaktionsrohres wurden mit unter Druck befindlichem Wasserdampf auf 220°C gehalten.

3. Die Gasphasenoxidation

Das vorstehend beschriebene erste Reaktionsrohr wurde mit einem Reaktionsgasausgangsgemisch der nachfolgenden Zusammensetzung kontinuierlich beschickt, wobei die Belastung und die Thermostatisierung des ersten Reaktionsrohres variiert wurden:

| | |
|---|---|
| 6 | bis 6,5 Vol-% Propen, |
| 3 | bis 3,5 Vol-% $H_2O$, |
| 0,3 | bis 0,5 Vol-% CO, |
| 0,8 | bis 1,2 Vol-% $CO_2$, |
| 0,025 | bis 0,04 Vol-% Acrolein, |
| 10,4 | bis 10,7 Vol-% $O_2$ und |
| | als Restmenge ad 100 % molekularer Stickstoff. |

Dem Produktgasgemisch der ersten Reaktionsstufe wurde am Ausgang des ersten Reaktionsrohres eine kleine Probe für eine gaschromatographische Analyse entnommen. Im übrigen wurde das Produktgasgemisch unter Zudüsen von eine Temperatur von 25°C aufweisender Luft unmittelbar in die nachfolgende Acroleino- xidationsstufe (zu Acrylsäure) geführt (Reaktionsstufe 2). Vom Produktgasgemisch der Acroleinoxidationsstufe wurde ebenfalls eine kleine Probe für eine gaschromatographische Analyse entnommen. Im übrigen wurde die Acrylsäure vom Produktgasgemisch der zweiten Reaktionsstufe in an sich bekannter Weise abgetrennt und ein Teil des verbleibenden Restgases zur Beschickung der Propenoxidationsstufe wiederverwendet (als sogenanntes Kreisgas), was den Acroleingehalt des vorgenannten Beschickungsgases und die geringe Varianz der Feedzusammensetzung erklärt.

Der Druck am Eingang des ersten Reaktionsrohres variierte in Abhängigkeit von der gewählten Propenbelastung im Bereich von 3,0 bis 0,9 bar. Am Ende der Reaktionszonen A, C befand sich ebenfalls eine Analysenstelle. Der Druck am Eingang des zweiten Reaktionsrohres variierte in Abhängigkeit von der Acroleinbelastung im Bereich von 2 bis 0,5 bar.

Die für die verschiedenen Katalysatorbeschichtungen in Abhängigkeit von den gewählten Belastungen und der gewählten Aluminium-Thermostatisierung sowie der praktizierten Luftzugabe (nach der ersten Reaktionsstufe) erzielten Ergebnisse zeigen. die nachfolgenden Tabellen (vorstehende Beispiele können erfindungsgemäß in entsprechender Weise (d.h., z.B. bei gleicher Last) auch in Einzonenrohreaktoren durchgeführt werden; die Temperatur in der ersten Stufe sollte dann zweckmäßig 320 bis 360°C und in der zweiten Stufe 245 bis 275°C betragen; diesbezüglich ist es ferner empf ehlenswert, in der ersten Reaktionsstufe die 300 cm Beschickung mit Festbettkatalysator 1 durch nachfolgende, sich nur noch auf eine Länge von 270 cm erstreckende, Beschickung zu ersetzen:

in Strömungsrichtung zunächst auf einer Länge von 100 cm ein Gemisch aus 65 Vol.-% Festbettkatalysator 1 und 35 Vol.-% Steatitringen (Außendurchmesser x Innendurchmesser x Länge = 5 mm x 3 mm x 2 mm) und daran anschließend auf einer Länge von 170 cm ein Gemisch aus 90 Vol.-% Festbettkatalysator 1 und 10 Vol.-% der vorstehenden Steatitringe;

außerdem empfiehlt es sich diesbezüglich in der zweiten Reaktionsstufe die 300 cm Beschickung mit Festbettkatalysator 2 durch nachfolgend Beschickung entsprechender Länge zu ersetzten:

in Strömungsrichtung zunächst auf einer Länge von 100 cm ein Gemisch aus 70 Vol.-% Festbettkatalysator 2 und 30 Vol.-% Steatitringen (Außendurchmesser x Innendurchmesser x Länge = 7 mm x 3 mm x 4 mm) und daran anschließend 200 cm reiner Festbettkatalysator 2;).

$T_A$, $T_B$, $T_C$, $T_D$ stehen für die Temperatur der Aluminiumblöcke in den Reaktionszonen A, B, C und D.

$U_{PA}$ ist der Propenumsatz am Ende der Reaktionszone A.

$U_{PB}$ ist der Propenumsatz am Ende der Reaktionszone B.

$S_{WP}$ ist die Selektivität der Acroleinbildung sowie der Acrylsäurenebenproduktbildung zusammengenommen nach der ersten Reaktionsstufe und bezogen auf umgesetztes Propen.

$U_{AC}$ ist der Acroleinumsatz am Ende der Reaktionszone C.

$U_{AD}$     ist der Acroleinumsatz am Ende der Reaktionszone D.

$U_{PD}$     ist der Propenumsatz am Ende der Reaktionszone D.

$S_{AS}$     ist die Selektivität der Acrylsäurebildung nach der zweiten Reaktionsstufe und bezogen auf umgesetztes Propen.

$RZA_{AS}$   ist die Raum-Zeit-Ausbeute an Acrlysäure am Ausgang des zweiten Reaktionsrohres.

V       ist das molare Verhältnis von molekularem Sauerstoff:Acrolein im Reaktionsgasausgangsgemisch 2

M       ist die nach der ersten Reaktionsstufe zugedüste Menge an Luft.

Tabelle 1

Katalysatorbeschickung: erfindungsgemäßer Festbettkatalysator 1 / erfindungsgemäßer
Festbettkatalysator 2

| Propenbelastung [Nl Propen/l·h] | $T_A$ [°C] | $T_B$ [°C] | $U_{PA}$ (%) | $U_{PB}$ (%) | $S_{WP}$ (%) | M (Nl/h) | V |
|---|---|---|---|---|---|---|---|
| 160 | 319 | 329 | 69,3 | 94,5 | 94,4 | 420 | 1,39 |
| 175 | 325 | 336 | 74,8 | 94,55 | 95,5 | 520 | 1,49 |
| 200 | 333 | 344 | 72,7 | 94,4 | 95,5 | 640 | 1,54 |

| Acroleinbelastung [Nl Acrolein/l·h] | $T_C$ [°C] | $T_D$ [°C] | $U_{AC}$ (%) | $U_{AD}$ (%) | $U_{PD}$ (%) | $S_{AS}$ (%) | $RZA_{AS}$ (g/l·h) |
|---|---|---|---|---|---|---|---|
| 139 | 255 | 276 | 50,9 | 99,2 | 94,4 | 95,2 | 228 |
| 152 | 261 | 277 | 77,3 | 99,2 | 94,5 | 95,8 | 255 |
| 173 | 261 | 281 | 72,6 | 99,3 | 94,4 | 95,7 | 291 |

Tabelle 2

Katalysatorbeschickung:Vergleichsfestbettschalenkatalysator 1 / Vergleichsfestbettschalenkatalysator 2

| Propenbelastung [Nl Propen/l·h] | $T_A$ [°C] | $T_B$ [°C] | $U_{PA}$ (%) | $U_{PB}$ (%) | $S_{WP}$ (%) | M (Nl/h) | V |
|---|---|---|---|---|---|---|---|
| 160 | 342 | 366 | 65,2 | 93,4 | 93,1 | 410 | 1,47 |
| 175 | 350 | 380 | 73,1 | 93,0 | 92,9 | 515 | 1,52 |
| 200 | 360 | 385 | 69,7 | 92,8 | 92,5 | 645 | 1,59 |

| Acroleinbelastung [Nl Acrolein/l·h] | $T_C$ [°C] | $T_D$ [°C] | $U_{AC}$ (%) | $U_{AD}$ (%) | $U_{PD}$ (%) | $S_{AS}$ (%) | $RZA_{AS}$ (g/l·h) |
|---|---|---|---|---|---|---|---|
| 130 | 255 | 268 | 59,1 | 99,1 | 93,4 | 94,3 | 206 |
| 141 | 263 | 279 | 67,2 | 98,9 | 93,0 | 94,0 | 220 |
| 159,6 | 265 | 285 | 69,1 | 98,7 | 92,8 | 93,8 | 248 |

Tabelle 3

Katalysatorbeschickung: Vergleichsfestbettvollzylinderkatalysator 1 / Vergleichsfestbettvollzylinderkatalysator 2

| Propenbelastung [Nl Propen/l·h] | $T_A$ [°C] | $T_B$ [°C] | $U_{PA}$ (%) | $U_{PB}$ (%) | $S_{WP}$ (%) | M (Nl/h) | V |
|---|---|---|---|---|---|---|---|
| 160 | 304 | 310 | 63,4 | 94,3 | 89,2 | 470 | 1,47 |
| 175 | 308 | 315 | 69,3 | 94,3 | 90,1 | 550 | 1,56 |
| 200 | 310 | 320 | 68,2 | 94,4 | 89,8 | 700 | 1,64 |

| Acroleinbelastung [Nl Acrolein/l·h] | $T_C$ [°C] | $T_D$ [°C] | $U_{AC}$ (%) | $U_{AD}$ (%) | $U_{PD}$ (%) | $S_{AS}$ (%) | $RZA_{AS}$ (g/l·h) |
|---|---|---|---|---|---|---|---|
| 131 | 250 | 259 | 72,3 | 99,3 | 94,3 | 93,1 | 205 |
| 142 | 254 | 263 | 67,3 | 99,3 | 94,3 | 93,3 | 223 |
| 160 | 258 | 267 | 69,2 | 99,3 | 94,4 | 92,8 | 250 |

**Patentansprüche**

1. Verfahren der katalytischen Gasphasenoxidation von Propen zu Acrylsäure, bei dem man ein Propen, molekularen Sauerstoff und wenigstens ein Inertgas enthaltendes Reaktionsgasausgangsgemisch 1, das den molekularen Sauerstoff und das Propen in einem molaren Verhältnis $O_2 : C_3H_6 \geq 1$ enthält, zunächst in einer ersten Reaktionsstufe bei erhöhter Temperatur so über einen ersten Festbettkatalysator, dessen Aktivmasse wenigstens ein Molybdän und/oder Wolfram sowie Wismut, Tellur, Antimon, Zinn und/oder Kupfer enthaltendes Multimetalloxid ist, führt, daß der Propenumsatz bei einmaligem Durchgang $\geq 90$ mol-% und die damit einhergehende Selektivität der Acroleinbildung sowie der Acrylsäurenebenproduktbildung zusammengenommen $\geq 90$ mol-% betragen, die Temperatur des die erste Reaktionsstufe verlassenden Produktgasgemisches durch direkte und/oder indirekte Kühlung gegebenenfalls verringert und dem Produktgasgemisch gegebenenfalls molekularen Sauerstoff und/oder Inertgas zugibt, und danach das Produktgasgemisch als Acrolein, molekularen Sauerstoff und wenigstens ein Inertgas enthaltendes Reaktionsgasausgangsgemisch 2, das den molekularen Sauerstoff und das Acrolein in einem molaren Verhältnis $O_2 : C_3H_4O \geq 0,5$ enthält, in einer zweiten Reaktionsstufe bei erhöhter Temperatur so über einen zweiten Festbettkatalysator, dessen Aktivmasse wenigstens ein Molybdän und Vanadin enthaltendes Multimetalloxid ist, führt, daß der Acroleinumsatz bei einmaligem Durchgang $\geq 90$ mol-% und die Selektivität der über beide Reaktionsstufen bilanzierten Acrylsäurebildung, bezogen auf umgesetztes Propen, $\geq 80$ mol-% beträgt, das **dadurch gekennzeichnet ist, daß**

   a) die Belastung des ersten Festbettkatalysators mit dem im Reaktionsgasausgangsgemisch 1 enthaltenen Propen $\geq 160$ Nl Propen/l Katalysatorschüttung· h beträgt,

   b) die Belastung des zweiten Festbettkatalysators mit dem im Reaktionsgasausgangsgemisch 2 enthaltenen Acrolein $\geq 140$ Nl Acrolein/l Katalysatorschüttung · h beträgt und

   c) sowohl die Geometrie der Katalysatorformkörper des ersten Festbettkatalysators als auch die Geometrie der Katalysatorformkörper des zweiten Festbettkatalysators mit der Maßgabe ringförmig ist, daß

   - der Ringaußendurchmesser 2 bis 11 mm,
   - die Ringlänge 2 bis 11 mm und
   - die Wandstärke des Ringes 1 bis 5 mm beträgt.

2. Verfahren der katalytischen Gasphasenoxidation von Propen zu Acrolein und/oder Acrylsäure, bei dem man ein Propen, molekularen Sauerstoff und wenigstens ein Inertgas enthaltendes Reaktionsgasausgangsgemisch 1, das den molekularen Sauerstoff und das Propen in einem molaren Verhältnis $O_2 : C_3H_6 \geq 1$ enthält, in einer Reaktionsstufe bei erhöhter Temperatur so über einen ersten Festbettkatalysator, dessen Aktivmasse wenigstens ein Molybdän und/oder Wolfram sowie Wismut, Tellur, Antimon, Zinn und/oder Kupfer enthaltendes Multimetalloxid ist, führt, daß der Propenumsatz bei einmaligem Durchgang $\geq 90$ mol-% und die damit einhergehende Selektivität der Acroleinbildung sowie der Acrylsäurenebenproduktbildung zusammengenommen $\geq 90$ mol-% betragen, das **dadurch gekennzeichnet ist, daß**

   a) die Belastung des Festbettkatalysators mit dem im Reaktionsgasausgangsgemisch 1 enthaltenen Propen $\geq 160$ Nl Propen/l Katalysatorschüttung · h beträgt und

   b) die Geometrie der Katalysatorformkörper des Festbettkatalysators mit der Maßgabe ringförmig ist, daß

   - der Ringaußendurchmesser 2 bis 11 mm,
   - die Ringlänge 2 bis 11 mm und
   - die Wandstärke des Ringes 1 bis 5 mm beträgt.

3. Verfahren nach Anspruch 1, **dadurch gekennzeichnet, dass** das Reaktionsgasausgangsgemisch 2 den molekularen Sauerstoff und das Acrolein in einem molaren Verhältnis $O_2:C_3H_4O \geq 1$ enthält.

4. Verfahren nach einem der Ansprüche 1 bis 3, **dadurch gekennzeichnet, dass** als erster Festbettkatalysator eine Katalysatorschüttung 1 verwendet wird, deren volumenspezifische Aktivität in Strömungsrichtung des Reaktionsgasausgangsgemisches 1 durch Verdünnung mit Inertmaterial kontinuierlich, abrupt oder stufenförmig zunimmt.

5. Verfahren nach Anspruch 1 oder einem der Ansprüche 3 oder 4, **dadurch gekennzeichnet, dass** als zweiter Festbettkatalysator eine Katalysatorschüttung 2 verwendet wird, deren volumenspezifische Aktivität in Strömungsrichtung des Reaktionsgasgemisches durch Verdünnung mit Inertmaterial kontinuierlich, abrupt oder stufenförmig zunimmt.

6. Verfahren nach einem der Ansprüche 1 bis 5, **dadurch gekennzeichnet, dass** die Durchführung der ersten Reaktionsstufe in einem Zweizonenrohrbündelreaktor erfolgt.

7. Verfahren nach Anspruch 1 oder einem der Ansprüche 3 bis 6, **dadurch gekennzeichnet, dass** die Durchführung der zweiten Reaktionsstufe in einem Zweizonenrohrbündelreaktor erfolgt.

**Claims**

1. A process for the catalytic gas-phase oxidation of propene to acrylic acid, in which a reaction gas starting mixture 1 which comprises propene, molecular oxygen and at least one inert gas and contains the molecular oxygen and the propene in a molar $O_2 : C_3H_6$ ratio of $\geq 1$ is first passed, in a first reaction stage at elevated temperatures, over a first fixed-bed catalyst whose active material is at least one multimetal oxide containing molybdenum and/or tungsten and bismuth, tellurine, antimony, tin and/or copper, in such a way that the propene conversion in a single pass is $\geq 90$ mol% and the associated selectivity of the acrolein formation and of the acrylic acid byproduct formation together is $\geq 90$ mol%, the temperature of the product gas mixture leaving the first reaction stage is, if required, reduced by direct and/or indirect cooling and, if required, molecular oxygen and/or inert gas are added to the product gas mixture, and the product gas mixture, as reaction gas starting mixture 2 which comprises acrolein, molecular oxygen and at least one inert gas and contains the molecular oxygen and the acrolein in a molar $O_2 : C_3H_4O$ ratio of $\geq 0.5$, is then passed, in a second reaction stage at elevated temperatures, over a second fixed-bed catalyst whose active material is at least one multimetal oxide containing molybdenum and vanadium, in such a way that the acrolein conversion in a single pass is $\geq 90$ mol% and the selectivity of the acrylic acid formation balanced over both reaction stages is $\geq 80$ mol%, based on propene converted, wherein

   a) the loading of the first fixed-bed catalyst with the propene contained in the reaction gas starting mixture 1 is $\geq 160$ l (S.T.P.) of propene/l of catalyst bed · h,

   b) the loading of the second fixed-bed catalyst with the acrolein contained in the reaction gas starting mixture 2 is $\geq 140$ l (S.T.P.) of acrolein/l of catalyst bed l · h and

   c) both the geometry of the catalyst moldings of the first fixed-bed catalyst and the geometry of the catalyst moldings of the second fixed-bed catalyst are annular, with the proviso that

   -   the external ring diameter is from 2 to 11 mm,
   -   the ring length is from 2 to 11 mm and
   -   the wall thickness of the ring is from 1 to 5 mm.

2. A process for the catalytic gas-phase oxidation of propene to acrolein and/or acrylic acid, in which a reaction gas starting mixture 1 which comprises propene, molecular oxygen and at least one inert gas and contains the molecular oxygen and the propene in a molar $O_2 : C_3H_6$ ratio of $\geq 1$ is passed, in a reaction stage at elevated temperatures, over a first fixed-bed catalyst whose active material is at least one multimetal oxide containing molybdenum and/or tungsten and bismuth, tellurium, antimony, tin and/or copper, in such a way that the propene conversion in a single pass is $\geq 90$ mol% and the associated selectivity of the acrolein formation and of the acrylic acid byproduct formation together is $\geq 90$ mol%, wherein

   a) the loading of the fixed-bed catalyst with the propene contained in the reaction gas starting mixture 1 is $\geq 160$ l (S.T.P.) of propene/l of the catalyst bed · h and

   b) the geometry of the catalyst moldings of the fixed-bed catalyst is annular, with the proviso that

   -   the external diameter of the ring is from 2 to 11 mm,
   -   the ring length is from 2 to 11 mm and
   -   the wall thickness of the ring is from 1 to 5 mm.

3. A process as claimed in claim 1, wherein the reaction gas starting mixture 2 contains the molecular oxygen and the acrolein in a molar $O_2:C_3H_4O$ ratio of $\geq 1$.

4. A process as claimed in any of claims 1 to 3, wherein a catalyst bed 1 whose volume-specific activity increases continuously, abruptly or stepwise in the direction of flow of the reaction gas starting mixture 1 through dilution with inert material is used as the first fixed-bed catalyst.

5. A process as claimed in claim 1 or either of claims 3 and 4, wherein a catalyst bed 2 whose volume-specific activity increases continuously, abruptly or stepwise in the direction of flow of the reaction gas mixture through dilution with inert material is used as the second fixed-bed catalyst.

6. A process as claimed in any of claims 1 to 5, wherein the first reaction stage is carried out in a two-zone tube-bundle reactor.

7. A process as claimed in claim 1 or any of claims 3 to 6, wherein the second reaction stage is carried out in a two-zone tube-bundle reactor.

**Revendications**

1. Procédé d'oxydation catalytique en phase gazeuse de propène pour former de l'acide acrylique, dans lequel on guide un mélange de départ de gaz réactionnels 1 qui contient du propène, de l'oxygène moléculaire et au moins un gaz inerte et qui contient l'oxygène moléculaire et le propène dans un rapport molaire de $O_2/C_3H_6 \geq 1$, tout d'abord dans une première étape réactionnelle à température élevée en passant par un premier catalyseur à lit fixe, dont la masse active est au moins un oxyde multimétallique contenant du molybdène et/ou du tungstène ainsi que du bismuth, du tellure, de l'antimoine, de l'étain et/ou du cuivre, de façon que la conversion du propène soit, pour un seul passage, $\geq 90$ moles % et la sélectivité ainsi développée de la formation d'acroléine ainsi que de la formation du produit secondaire acide acrylique soient conjointement $\geq 90$ moles %, on réduit éventuellement la température du mélange de gaz produit quittant la première étape réactionnelle par un refroidissement direct et/ou indirect et on ajoute au mélange de gaz produit éventuellement de l'oxygène moléculaire et/ou du gaz inerte, et ensuite on guide le mélange de gaz produit sous la forme d'un mélange de départ de gaz réactionnels 2 qui contient de l'acroléine, de l'oxygène moléculaire et au moins un gaz inerte et qui contient l'oxygène moléculaire et l'acroléine dans un rapport molaire de $O_2/C_3H_4O \geq 0,5$, dans une deuxième étape réactionnelle à température élevée en passant par un deuxième catalyseur à lit fixe, dont la masse active est au moins un oxyde multimétallique contenant du molybdène et du vanadium, de façon que la conversion d'acroléine soit, pour un passage unique, $\geq 90$ moles % et la sélectivité de la formation d'acide acrylique, dont la balance a été établie sur les deux étapes réactionnelles, par rapport au propène mis à réagir, est $\geq 80$ moles %, **caractérisé en ce que**

a) la charge du premier catalyseur à lit fixe par le propène contenu dans le mélange de départ de gaz réactionnels 1 est $\geq 160$ Nl de propène/l de masse de catalyseur·h,
b) la charge du deuxième catalyseur à lit fixe par l'acroléine contenue dans le mélange de départ de gaz réactionnels 2 est $\geq 140$ Nl d'acroléine/l de masse de catalyseur·h, et
c) aussi bien la géométrie des corps façonnés du premier catalyseur à lit fixe que la géométrie des corps façonnés du deuxième catalyseur à lit fixe sont annulaires à la condition que

- le diamètre externe d'anneau soit de 2 à 11 mm,
- la longueur d'anneau de 2 à 11 mm et
- l'épaisseur de paroi de l'anneau de 1 à 5 mm.

2. Procédé d'oxydation catalytique en phase gazeuse de propène pour former de l'acroléine et/ou de l'acide acrylique, dans lequel on guide un mélange de départ de gaz réactionnels 1 qui contient du propène, de l'oxygène moléculaire et au moins un gaz inerte et qui contient l'oxygène moléculaire et le propène dans un rapport molaire de $O_2/C_3H_6 \geq 1$, dans une étape réactionnelle à température élevée en passant par un premier catalyseur à lit fixe, dont la masse active est au moins un oxyde multimétallique contenant du molybdène et/ou du tungstène ainsi que du bismuth, du tellure, de l'antimoine, de l'étain et/ou du cuivre, de façon que la conversion de propène soit, pour un passage unique, $\geq 90$ moles % et la sélectivité ainsi développée de la formation d'acroléine ainsi que de la formation du produit secondaire acide acrylique soient conjointement $\geq 90$ moles %, **caractérisé en ce que**

a) la charge du catalyseur à lit fixe par le propène contenu dans le mélange de départ de gaz réactionnels 1 est ≥ 160 Nl de propène/l de masse de catalyseur.h, et

b) la géométrie des corps façonnés du catalyseur à lit fixe est annulaire à la condition que

- le diamètre externe d'anneau soit de 2 à 11 mm,
- la longueur d'anneau de 2 à 11 mm et
- l'épaisseur de paroi de l'anneau de 1 à 5 mm.

**3.** Procédé suivant la revendication 1, **caractérisé en ce que** le mélange de départ de gaz réactionnels 2 contient l'oxygène moléculaire et l'acroléine dans un rapport molaire de $O_2/C_3H_4O \geq 1$.

**4.** Procédé suivant l'une des revendications 1 à 3, **caractérisé en ce que**, comme premier catalyseur à lit fixe, on utilise une masse de catalyseur 1 dont l'activité spécifique en volume augmente de manière continue, abrupte ou par gradins dans le sens d'écoulement du mélange de départ de gaz réactionnels 1 par dilution par de la matière inerte.

**5.** Procédé suivant la revendication 1 ou une des revendications 3 et 4, **caractérisé en ce que**, comme deuxième catalyseur à lit fixe, on utilise une masse de catalyseur 2 dont l'activité spécifique en volume augmente de manière continue, abrupte ou par gradins dans le sens d'écoulement du mélange de gaz réactionnels par dilution par de la matière inerte.

**6.** Procédé suivant l'une des revendications 1 à 5, **caractérisé en ce que** la mise en oeuvre de la première étape réactionnelle a lieu dans un réacteur à faisceau tubulaire en deux zones.

**7.** Procédé suivant la revendication 1 ou une des revendications 3 à 6, **caractérisé en ce que** la mise en oeuvre de la deuxième étape réactionnelle a lieu dans un réacteur à faisceau tubulaire en deux zones.